(19) Europäisches Patentamt
European Patent Office
Office européen des brevets

(11) EP 3 542 719 B1

(12) **EUROPEAN PATENT SPECIFICATION**

(45) Date of publication and mention of the grant of the patent:
**25.01.2023 Bulletin 2023/04**

(21) Application number: **16921827.8**

(22) Date of filing: **16.11.2016**

(51) International Patent Classification (IPC):
***A61B 5/11*** *(2006.01)* ***A61B 5/00*** *(2006.01)*

(52) Cooperative Patent Classification (CPC):
**A61B 5/11; A61B 5/1114; A61B 5/1116; A61B 5/112; A61B 5/1122; A61B 5/1123; A61B 5/7278;** A61B 5/6828; A61B 5/6829; A61B 2562/0219

(86) International application number:
**PCT/JP2016/083996**

(87) International publication number:
**WO 2018/092219 (24.05.2018 Gazette 2018/21)**

(54) **INFORMATION PROCESSING SYSTEM, INFORMATION PROCESSING DEVICE AND INFORMATION PROCESSING METHOD**

INFORMATIONSVERARBEITUNGSSYSTEM, INFORMATIONSVERARBEITUNGSVORRICHTUNG UND INFORMATIONSVERARBEITUNGSVERFAHREN

SYSTÈME DE TRAITEMENT D'INFORMATIONS, DISPOSITIF DE TRAITEMENT D'INFORMATIONS, ET PROCÉDÉ DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.09.2019 Bulletin 2019/39**

(73) Proprietor: **Fujitsu Limited**
**Kawasaki-shi, Kanagawa 211-8588 (JP)**

(72) Inventors:
- **SASAMOTO, Yuki**
**Kawasaki-shi**
**Kanagawa 211-8588 (JP)**
- **HOTTA, Shinji**
**Kawasaki-shi**
**Kanagawa 211-8588 (JP)**
- **INOMATA, Akihiro**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**

(56) References cited:
**JP-A- 2013 106 768**
**JP-A- 2016 112 108**
**US-A1- 2004 064 286**
**US-A1- 2016 018 225**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 542 719 B1

**Description**

TECHNICAL FIELD

The present invention relates to an information processing system, an information processing apparatus and an information processing method.

BACKGROUND ART

For example, the number of cases has been increased where care for patients made only in hospitals is made at patient's home and a nursing care facility in each region. In addition, a person in charge of care for the patient is required to recognize a state of the patient and the course (change) of the patient even in a case where the patient is at home, a nursing care facility, and the like.

As the related technique, a technique has been proposed for determining a relative direction of a device with respect to a body by determining a conversion relation between a body reference system and a reference system regarding a direction detection unit (for example JP 2013-532539 A). A technique has been proposed for detecting a rotation angle around a center point of a behavior detection device and correcting acceleration data based on the detection result (for example JP 2005-172625 A). A technique has been proposed for determining a walk balance based on a difference between a sum of magnitudes of forward vectors and a sum of magnitudes of backward vectors calculated from acceleration vector data (for example WO 2007/052631 A).

In addition, as the related technique, a technique for detecting walking by using a gyro sensor and quantifying a walking speed, a stride length, and the like has been proposed (for example Fraccaro et al., "Real-world Gyroscopebased Gait Event Detection and Gait Feature Extraction". eTELEMED 2014). A technique for detecting seating and standing up by using an acceleration sensor and a gyro sensor has been proposed (for example Van Lummel et al., "Automated approach for quantifying the repeated sit-to-stand using one body fixed sensor in young and older adults". Gait & Posture. Vol.38, pp.153-156, 2013). US 2004/0064286 A1 relates to dead reckoning navigation for persons on foot by use of accelerometers in a navigation device, which are to deduce when abnormal steps are being taken and modify navigation calculations accordingly. Running and walking sideways or backwards are among the situations which can be detected and compensated. US 2016/0018225 A1 relates to methods and systems for determining a moving direction of a mobile device user. The methods and systems relate to using an inertial navigation system such as an accelerometer and gyroscope to aid in the determination of the moving direction of the user.

SUMMARY OF INVENTION

TECHNICAL PROBLEM

For example, it is considered that a motion of a person to be measured (for example, patient) is detected by using a wearable motion sensor and the detected motion is quantified. However, in a case where an attachment position of the motion sensor is different from an expected position, accuracy of quantification of the motion is deteriorated.

As one aspect, an object of the present invention is to suppress deterioration in accuracy of quantification of a motion.

SOLUTION TO PROBLEM

The subject-matter of the independent claims solves the above technical problem whilst the dependent claims describe further preferred embodiments.

ADVANTAGEOUS EFFECTS OF INVENTION

According to one aspect, deterioration in accuracy of quantification of a motion can be suppressed.

BRIEF DESCRIPTION OF DRAWINGS

FIG. 1 is a diagram illustrating a direction of each axis.
FIG. 2 is a diagram illustrating an example of an overall configuration of a system according to an embodiment.
FIG. 3 is an example of motion data at the time of walking.

FIG. 4 is a diagram illustrating an example of an information processing apparatus according to a second embodiment.
FIG. 5 is a diagram illustrating an example of a data acquisition device.
FIG. 6 is a flowchart illustrating an example of a processing flow according to the embodiment.
FIG. 7 is a diagram illustrating an example of a motion section in the motion data at the time of walking.
FIG. 8 is a diagram illustrating an example of a determination result of a mixing degree.
FIG. 9 is a diagram illustrating an example of motion data before and after motion conversion.
FIG. 10 is a diagram illustrating an example of the motion data before and after the motion conversion.
FIG. 11 is a diagram illustrating an example of the motion data before and after the motion conversion.
FIG. 12 is a diagram illustrating an example of an information processing apparatus according to a second embodiment.
FIG. 13 is a flowchart (part 1) illustrating an example of a flow of the information processing apparatus according to the second embodiment.
FIG. 14 is a flowchart (part 2) illustrating the example of the flow of the information processing apparatus according to the second embodiment.
FIG. 15 illustrates examples of motion data at the time of walking when a sensor is fixed and is not fixed.
FIG. 16 is a diagram illustrating an example of motion data of a leg in a case where an attachment position gradually changes.
FIG. 17 is a diagram illustrating an example of a change in a mixing degree in a case where the attachment position gradually changes.
FIG. 18 is a diagram illustrating an example of motion data in a case where a motion for walking backward during walking is included.
FIG. 19 is a diagram illustrating motion data before and after conversion in a case where a conversion parameter is a local solution.
FIG. 20 is a diagram illustrating section separation of motion data including an abnormal motion.
FIG. 21 is a diagram illustrating an example of motion data of a direction change after the conversion in the plurality of motion sections.
FIG. 22 is a diagram illustrating motion data after conversion of the direction change.
FIG. 23 is a diagram illustrating rotation directions of a leg.
FIG. 24 is motion data from each sensor obtained by an extraction unit according to a first example.
FIG. 25 is a diagram illustrating an example of a motion extraction algorithm.
FIG. 26 is a diagram illustrating an example of an extracted motion section.
FIG. 27 is a diagram illustrating an example of a determination index of the mixing degree.
FIG. 28 is a diagram illustrating an example of information stored in a state conversion amount DB.
FIG. 29 is a diagram illustrating an example of motion data before and after the conversion according to the first example.
FIG. 30 is a diagram illustrating an example of a motion feature amount regarding walking stored in a feature amount DB.
FIG. 31 is motion data from each sensor 4 obtained by an extraction unit 12 according to a second example.
FIG. 32 is a diagram illustrating an example of motion data before and after conversion according to the second example.
FIG. 33 is a diagram illustrating an example of a motion feature amount regarding a direction change stored in the feature amount DB.
FIG. 34 is a diagram for explaining an exemplary hardware configuration of an information processing apparatus.

## DESCRIPTION OF EMBODIMENTS

Hereinafter, embodiments will be described with reference to the drawings. Each axis used in the description of the present embodiment will be described. FIG. 1 is a diagram illustrating directions of respective axes with reference to a person to be measured. The description according to the present embodiment will be made based on the axes illustrated in FIG. 1. The person to be measured is, for example, a patient and a care recipient.

A frontal plane is an arbitrary plane that divides a body of the person to be measured into a ventral and dorsal portions. A horizontal plane is a plane perpendicular to the direction of gravity. A sagittal plane is a plane that passes through the center of the body of the person to be measured and is parallel to a plane that divides the body into a left and right portion.

A frontal-horizontal axis is an axis orthogonal to the sagittal plane. A sagittal-horizontal axis is an axis orthogonal to the frontal plane. A vertical axis is an axis orthogonal to the horizontal plane.

In FIG. 1, an origin where the axes intersect with each other is positioned near the center of the body. However, the position of the origin is not limited to the position illustrated in FIG. 1. For example, a case where motion data indicating walking is obtained, the position of the origin is near the foot.

In the description of the present embodiment, it is assumed that motion data of a rotation direction around the frontal-horizontal axis be X'(t). It is assumed that motion data of a rotation direction around the vertical axis be Y'(t). It is assumed that motion data of a rotation direction around the sagittal-horizontal axis be Z'(t).

The motion data in the present embodiment indicates an angular speed per unit time in the rotation direction around each axis. In the description of the present embodiment, in a case where the directions of the motion data are not distinguished from each other, the motion data is simply described as motion data. The motion data may be data other than the angular speed (for example, speed).

<Example of Overall Configuration of System According to Embodiment>

FIG. 2 illustrates an example of an overall configuration of a system according to an embodiment. As illustrated in the example in FIG. 2, an information processing apparatus 1 is connected to a plurality of relay devices 3 via a network 2 The relay device 3 is connected to a plurality of sensors 4. The number of relay devices 3 and sensors 4 may be one.

The information processing apparatus 1 obtains data indicating motion measured by the sensor 4 (referred to as motion data below) via the network 2 and the relay device 3. The information processing apparatus 1 is placed, for example, in a hospital. The information processing apparatus 1 is an example of a computer.

The network 2 is, for example, a wide area communication network and is used for communication between the information processing apparatus 1 and the relay device 3.

The relay device 3 is placed, for example, in a patient's home or a nursing care facility. The relay device 3 relays communication between the network 2 and the sensor 4.

The sensor 4 is, for example, a motion sensor for measuring a movement and is attached to a hand, foot, and the like of the person to be measured. The sensor 4 is, for example, a gyro sensor for measuring an angular speed or an inertial sensor such as an acceleration sensor. The sensor 4 may include a plurality of types of sensors.

The configuration of the system according to the present embodiment is not limited to the configuration illustrated in FIG. 2. For example, it is possible that the system according to the present embodiment does not include the relay device 3. In that case, for example, the information processing apparatus 1 may obtain the motion data from the sensor 4 via the network 2.

Furthermore, the system according to the present embodiment may include a device (for example, server) that receives the motion data from the sensor 4 and transmits the received motion data to the relay device 3.

As assuming that the sensor 4 be attached to a specific position of the person to be measured, the information processing apparatus 1 according to the embodiment quantifies a motion by using an algorithm corresponding to a specific type of motion.

For example, in a case where the type of the motion is walking, by measuring a rotation speed in the frontal-horizontal axis direction during walking, a speed, a timing, and the like of stepping, landing, and leaving from the ground can be quantified. In addition, in a case where the type of the motion is to stand up, by measuring a rotation speed and an acceleration change in the frontal-horizontal axis direction at the time of standing up, a speed, a timing, an attitude change, and the like at the time of leaning forward can be quantified.

However, in a case where the motion is quantified as assuming that the sensor 4 is attached to the specific position, if the attachment position of the sensor 4 is changed from an expected position (normal position), there is a case where accuracy in quantification of the motion is deteriorated.

For example, if an initial attachment position of the sensor 4 is changed from the expected position, the accuracy in the quantification of the motion is deteriorated. Furthermore, a movement of the person to be measured who wears the sensor 4 may change the attachment position of the sensor 4 from the expected position. In this case, the accuracy in the quantification of the motion is deteriorated.

FIG. 3 is an example of motion data at the time of walking. FIG. 3(a) is motion data in a representative direction at the time of walking in a case where the sensor 4 is attached at a normal position of a leg of the person to be measured. FIG. 3(b) is motion data in the representative direction in a case where the sensor 4 is not attached at the normal position.

The representative direction is a rotation direction of any one of the axes illustrated in FIG. 1. In a case where the motion data for each of the plurality of directions is detected, the representative direction indicates a direction in which a motion feature amount of the motion data is equal to or more than a predetermined standard (first standard). The first standard may be arbitrarily set.

The number of representative directions is not limited to one and may be two or more. The motion feature amount used for specifying the representative direction is, for example, a value of a temporal change amount of the motion data. The motion feature amount used for specifying the representative direction may be, for example, an absolute value and a peak value of the temporal change amount or an area of a peak section.

In a case where the type of the motion is walking, usually, the representative direction is the rotation direction around the frontal-horizontal axis. In a case where the sensor 4 is not attached at the normal position, the angular speed around the frontal-horizontal axis is lower than that in a case where the sensor 4 is normally attached. Therefore, in the example illustrated in FIG. 3, an angular speed of motion data in Fig. 3(b) is lower than an angular speed of motion data in FIG. 3(a).

On the other hand, regardless of whether or not the sensor 4 is attached at the normal position, a motion of the person to be measured who wears the sensor 4 does not change. For example, regardless of whether or not the sensor 4 is attached at the normal position, usually, the person to be measured moves forward while rotating the legs at the time of walking. In addition, the person to be measured turns the body while rotating the leg at the time of a direction change. Therefore, walking has characteristics that a motion amount in the rotation direction around the frontal-horizontal axis increases.

The motion amount is, for example, a total value (integration value) of absolute values in a predetermined section of output values of the sensor 4.

Furthermore, for example, when the type of the motion is walking, there is a directionality that rotation in the rotation direction around the frontal-horizontal axis occurs. In addition, when the motion is the direction change, the motion has characteristics that rotation in the direction around the vertical axis occurs.

<Example of Information Processing Apparatus According to First Embodiment>

FIG. 4 is a diagram illustrating an example of an information processing apparatus according to a first embodiment. An information processing apparatus 1 includes a first storage unit 10, a first communication unit 11, an extraction unit 12, a mixing degree determination unit 13, a specification unit 14, a determination unit 15, a conversion unit 16, and a calculation unit 17.

The first storage unit 10 includes a motion extraction algorithm Data Base (DB) 10a, a determination index DB 10b, a state conversion amount DB 10c, and a feature amount DB 10d.

The first communication unit 11 receives motion data regarding a motion transmitted from the sensor 4.

The extraction unit 12 obtains the motion data from the sensor 4 via the first communication unit 11 and extracts motion data in a section in which a motion feature amount of the motion data is equal to or more than a threshold so as to extract data in a section in which a motion to be detected in the motion data is indicated.

The mixing degree determination unit 13 determines a mixing degree indicating the number of representative directions of the motion based on the motion data. For example, the mixing degree determination unit 13 determines the mixing degree based on variation in the motion data in each of three-dimensional directions.

The specification unit 14 specifies one or a plurality of moving directions (representative direction) in which the motion feature amount (first motion feature amount) is equal to or more than a first standard based on the motion data. It is preferable that the specification unit 14 specify one or a plurality of representative directions in which the motion feature amount is maximized. The specification unit 14 specifies the representative directions as many as the mixing degrees, for example, based on a magnitude of the motion feature amount in each direction of data obtained by rotationally converting the motion data.

The determination unit 15 determines a parameter used for conversion of the motion data based on a motion feature amount (second motion feature amount) in the representative direction specified by the specification unit 14. For example, the determination unit 15 determines the parameter used for the conversion of the motion data by using the specified representative direction so that the motion feature amount after the conversion in the representative direction specified by the specification unit 14 is equal to or more than a predetermined standard (second standard). For example, it is preferable that the determination unit 15 determine the parameter used for the conversion of the motion data so that the motion feature amount is maximized.

The conversion unit 16 converts the motion data by using the parameter determined by the determination unit 15.

The calculation unit 17 specifies the type of the motion based on the motion data converted by the conversion unit 16 and calculates (quantify) a feature amount of the motion.

<Example of Sensor>

FIG. 5 is a diagram illustrating an example of the sensor 4. The sensor 4 includes a second communication unit 41, a motion measurement unit 42, and a second storage unit 45. The second storage unit 45 includes a motion information DB 45a.

The second communication unit 41 transmits the motion data to the information processing apparatus 1. The motion measurement unit 42 measures a motion and makes the motion information DB 45a store the motion data indicating the motion. The motion information DB 45a stores the motion data generated by the motion measurement unit 42.

For example, the sensor 4 makes the motion information DB 45a temporarily store the motion data and periodically transmits the motion data to the information processing apparatus 1 via a relay device 3 and a network 2.

<Example of Processing Flow According to First Embodiment>

FIG. 6 is a flowchart illustrating an example of a processing flow according to the first embodiment. The first communication unit 11 receives the motion data indicating the motion measured by the sensor 4 (step S101).

The extraction unit 12 extracts data in a motion section indicating the motion to be detected from the received motion data (step S102). In a case where a previously specified type of the motion is set, the extraction unit 12 obtains an algorithm for extracting the motion section from data of the previously specified type of the motion from a motion extraction algorithm DB 10a before extracting the data in the section. In the present embodiment, it is assumed that the type of the motion "walking" be previously specified by a user.

For example, the extraction unit 12 calculates a motion feature amount of the motion data in each direction for each predetermined section and extracts data in a section in which the motion feature amount exceeds a threshold (extraction threshold) included in the algorithm. The motion feature amount used in step S102 is, for example, a composite value, a maximum value, a minimum value, or an average value of the motion data in each direction.

The composite value of the motion data is, for example, expressed by the following expression (1). In the expression (1), X(t), Y(t), and Z(t) indicate the motion amounts in the respective directions of the motion data. S(t) indicates a composite value of X(t), Y(t), and Z(t).

[Expression 1]

$$S(t) = \sqrt{(x(t))^2+(y(t))^2+(z(t))^2} \quad \cdots (1)$$

For example, the extraction unit 12 may extract a section in which a variance value, a period, or a peak value of the motion feature amount of the motion data in each direction exceeds the extraction threshold included in the algorithm.

The algorithm may include, for example, a specific motion pattern. Then, for example, the extraction unit 12 may compare the obtained motion data with the specific motion data pattern and extract the data in the section in which a difference is within a predetermined value.

In a case where the previously specified type of the motion is not set, the extraction unit 12 may extract data in a section in which the motion feature amount or the variance value, the period, or the peak value of the motion feature amount exceeds an extraction threshold that does not depend on the type of the motion.

FIG. 7 is a diagram illustrating an example of extraction of a motion section. FIG. 7(a) illustrates a composite value of motion data of straight walking. FIG. 7(b) illustrates a composite value of motion data of a direction change. As illustrated in FIG. 7, a section in which it is determined that a target motion has been performed (motion section) is specified by the extraction unit 12.

As illustrated in FIG. 6, the information processing apparatus 1 starts repetitive processing for each motion section extracted in step S102 (step S103). In step S103, when it is assumed that a reference N indicate the number of motion sections, the repetitive processing is performed in each motion section i (i = one to N). The numbers N and i are natural numbers.

The mixing degree determination unit 13 determines a mixing degree indicating the number of representative directions of the motion in the motion section i (step S104). For example, the mixing degree determination unit 13 obtains a determination index corresponding to a target type of the motion from the determination index DB 10b including the determination index of the mixing degree and determines the mixing degree based on the determination index. For example, by using main component analysis, the mixing degree determination unit 13 determines the mixing degree based on variation in the motion data in each of the three-dimensional directions.

In step S104, the mixing degree determination unit 13 may, for example, execute the main component analysis on the motion data in the target section and calculate a cumulative contribution ratio of the generated axis. Then, the mixing degree determination unit 13 uses the smallest number of axes among the number of axes of which the cumulative contribution ratio is equal to or more than a predetermined threshold (cumulative contribution ratio threshold) (for example, 0.9) as the mixing degree. The cumulative contribution ratio threshold is included in the determination index stored in the determination index DB 10b.

FIG. 8 is a diagram illustrating an example of a determination result of the mixing degree. An example illustrated in FIG. 8(a) (first example) illustrates the number of axes created by the main component analysis and the cumulative contribution ratio of the straight walking motion. In the example illustrated in FIG. 8(a), regarding all the number of axes, the cumulative contribution ratio exceeds the cumulative contribution ratio threshold (0.9). Therefore, the mixing degree determination unit 13 uses "one" which is the smallest number of axes from among the number of axes of which the cumulative contribution ratio exceeds the cumulative contribution ratio threshold as the mixing degree.

For example, in a case of straight walking, a main motion is to rotate the legs forward. Therefore, the motion amount in the rotation direction around the frontal-horizontal axis is large, and the rotation direction around the frontal-horizontal axis is used as the representative direction. On the other hand, the motion amounts in the rotation directions around the sagittal-horizontal axis and the vertical axis are small. That is, in the main component analysis, since the contribution ratio and variation in the rotation direction around the frontal-horizontal axis are large, and the contribution ratio and variation in the rotation directions around the sagittal-horizontal axis and the vertical axis are small, the mixing degree (the number of representative directions) is "one" according to the above processing.

An example illustrated in FIG. 8(b) (second example) illustrates the number of axes created by the main component analysis and the cumulative contribution ratio of the motion data in a case of the direction change. In the example illustrated in FIG. 8(b), in a case where the number of axes is two or three, the cumulative contribution ratio exceeds the cumulative contribution ratio threshold (0.9). Therefore, the mixing degree determination unit 13 uses "two" which is the smallest number of axes from among the number of axes of which the cumulative contribution ratio exceeds the cumulative contribution ratio threshold as the mixing degree.

For example, in a case of the direction change, since the person pivots while rotating the legs forward, the motion amounts in the rotation direction around the frontal-horizontal axis and the rotation direction around the vertical axis are large, and the rotation direction around the frontal-horizontal axis and the rotation direction around the vertical axis are used as the representative directions. On the other hand, the motion amount in the rotation direction around the sagittal-horizontal axis is small. That is, in the main component analysis, since the contribution ratio and variation in the rotation direction around the frontal-horizontal axis and the rotation direction around the vertical axis are large, and the contribution ratio and variation in the rotation directions around the sagittal-horizontal axis are small, the mixing degree (the number of representative directions) is "two" according to the above processing.

An example illustrated in FIG. 8(c) (third example) illustrates the number of axes created by the main component analysis and the cumulative contribution ratio of motion data in a case where the person steps up and down a spiral staircase. In the example illustrated in FIG. 8(c), in a case where the number of axes is three, the cumulative contribution ratio exceeds the cumulative contribution ratio threshold (0.9). Therefore, the mixing degree determination unit 13 uses "three" which is the smallest number of axes from among the number of axes of which the cumulative contribution ratio exceeds the cumulative contribution ratio threshold as the mixing degree.

For example, in a case where the person steps up and down the spiral staircase, the person pivots while rotating the legs forward, and a further motion in the vertical direction occurs. Therefore, the motion amounts in all the rotation directions around the frontal-horizontal axis, the vertical axis, and the sagittal-horizontal axis are large, and all the directions are used as the representative directions. That is, since the contribution ratio is large in all the directions in the main component analysis, the mixing degree (the number of representative directions) is "three" according to the above processing.

As illustrated in FIG. 6, the specification unit 14 specifies a representative direction in which the motion feature amount is equal to or more than the first standard based on the mixing degree (step S105). For example, the specification unit 14 may specify the representative direction, from among the plurality of directions, of which an order of the magnitude of the motion feature amount of the motion data is within a predetermined order. Alternatively, the first standard may be, for example, a predetermined threshold (first threshold). That is, the specification unit 14 specifies the direction, from among the plurality of directions, in which the motion feature amount is larger as the representative direction. The first standard can be preset.

In step S105, for example, if the mixing degree is one, the specification unit 14 specifies a single direction, from among the plurality of directions, in which the motion feature amount of the motion data maximized as the representative direction. For example, if the mixing degree is two, the specification unit 14 specifies two directions in which the total motion feature amount in two directions is maximized as the representative directions. For example, if the mixing degree is three, the specification unit 14 sets all the three directions as the representative directions.

In a case where the type of the motion has been set in advance, the specification unit 14 may specify the representative direction according to the type of the motion. For example, in a case where the type of the motion has been set to be the straight walking, the specification unit 14 specifies the rotation direction around the frontal-horizontal axis as the representative direction. Furthermore, for example, in a case where the type of the motion has been set to be the direction change, the specification unit 14 specifies the rotation direction around the frontal-horizontal axis and the rotation direction around the vertical axis as the representative directions. In addition, for example, in a case where the type of the motion has been set to be stepping up and down the spiral staircase, the specification unit 14 specifies all the directions as the representative directions.

For example, the determination unit 15 determines a parameter (conversion parameter) used for the conversion of the motion data so that the motion feature amount in the representative direction is equal to or more than the second standard (for example, maximum) (step S106).

The determination unit 15 determines, for example, a plurality of conversion parameter candidates. Then, the determination unit 15 converts the motion data by using the determined conversion parameter candidates and ranks the conversion parameters used for the converted motion data in descending order of the motion feature amount in the representative direction. Then, the determination unit 15 may determine, for example, the conversion parameters of which the order is within a predetermined order as the parameter used for conversion. Alternatively, the second standard may be, for example, a predetermined threshold (second threshold). That is, the determination unit 15 determines the conversion parameter so that the motion feature amount in the representative direction further increases. The second standard can be preset.

The motion feature amount used in step S106 is, for example, a value of a temporal change amount of the motion data, an absolute value or a peak value of the temporal change amount, or an area of a peak section.

Next, a specific example of the processing by the specification unit 14 and the determination unit 15 will be described. The converted motion data is represented by X'(t), Y'(t), and Z'(t) indicated in the expressions (2) to (4). Motion data X'(t), Y'(t), and Z'(t) indicated in the expressions (2) to (4) are motion data obtained by rotationally converting three-axes motion data X(t), Y(t), and Z(t) by using a conversion parameter θ = (α, β, γ).

[Expression 2]

$$X'(t) = (\cos(\alpha)\cos(\beta)\cos(\gamma) - \sin(\alpha)\sin(\gamma))X(t) + (-\sin(\alpha)\cos(\gamma) - \cos(\alpha)\cos(\beta)\sin(\gamma))Y(t) + \cos(\alpha)\sin(\beta)Z(t) \quad \cdots (2)$$

[Expression 3]

$$Y'(t) = (\sin(\alpha)\cos(\beta)\cos(\gamma) + \cos(\alpha)\sin(\gamma))X(t) + (\cos(\alpha)\cos(\gamma) - \sin(\alpha)\cos(\beta)\sin(\gamma))Y(t) + \sin(\alpha)\sin(\beta)Z(t) \quad \cdots (3)$$

[Expression 4]

$$Z'(t) = -\sin(\beta)\cos(\gamma)X(t) + \sin(\beta)\sin(\gamma)Y(t) + \cos(\beta)Z(t) \quad \cdots (4)$$

For example, in a case where the mixing degree is one, the specification unit 14 calculates evaluation functions $E_{11}$ to $E_{13}$ of the motion feature amount indicated in the following expressions (5) to (7) regarding each of the plurality of conversion parameter candidates θ = (α, β, γ). The specification unit 14 compares, for example, the maximum values of the evaluation functions $E_{11}$ to $E_{13}$.

Exression 5]

$$E_{11} = \sum_{t=1}^{T-1} |X'(t)| - \sum_{t=1}^{T-1} |Y'(t)| - \sum_{t=1}^{T-1} |Z'(t)| \quad \cdots \quad (5)$$

[Expression 6]

$$E_{12} = \sum_{t=1}^{T-1} |Y'(t)| - \sum_{t=1}^{T-1} |X'(t)| - \sum_{t=1}^{T-1} |Z'(t)| \quad \cdots \quad (6)$$

[Expression 7]

$$E_{13} = \sum_{t=1}^{T-1} |Z'(t)| - \sum_{t=1}^{T-1} |X'(t)| - \sum_{t=1}^{T-1} |Y'(t)| \quad \cdots \quad (7)$$

In a case where the maximum value of the evaluation function $E_{11}$ is the largest in the maximum values of the evaluation functions $E_{11}$ to $E_{13}$, the specification unit 14 specifies that X'(t) is the motion data in the representative direction. In this case, the determination unit 15 determines a conversion parameter of which the evaluation function $E_{11}$ is equal to or more than the second standard (for example, maximum) as the parameter used for conversion.

In a case where the maximum value of the evaluation function $E_{12}$ is the largest in the maximum values the evaluation functions $E_{11}$ to $E_{13}$, the specification unit 14 determines that Y'(t) is the motion data in the representative direction. In that case, the determination unit 15 determines a conversion parameter $\theta = (\alpha, \beta, \gamma)$ of which the evaluation function $E_{12}$ is equal to or more than the second standard (for example, maximum) as the parameter used for conversion.

In a case where the maximum value of the evaluation function $E_{13}$ is the largest in the maximum values of the evaluation functions $E_{11}$ to $E_{13}$, the specification unit 14 specifies that Z'(t) is the motion data in the representative direction. In that case, the determination unit 15 determines a conversion parameter $\theta = (\alpha, \beta, \gamma)$ of which the evaluation function $E_{13}$ is equal to or more than the second standard (for example, maximum) as the parameter used for conversion.

Furthermore, for example, in a case where the mixing degree is two, the specification unit 14 calculates evaluation values $E_{21}$ to $E_{23}$ indicated in the following expressions (8) to (10) regarding each of the plurality of conversion parameters $\theta = (\alpha, \beta, \gamma)$.

[Expression 8]

$$E_{21} = \sum_{t=1}^{T-1} |X'(t)| + \sum_{t=1}^{T-1} |Y'(t)| - \sum_{t=1}^{T-1} |Z'(t)| \quad \cdots \quad (8)$$

[Expression 9]

$$E_{22} = \sum_{t=1}^{T-1} |X'(t)| + \sum_{t=1}^{T-1} |Z'(t)| - \sum_{t=1}^{T-1} |Y'(t)| \quad \cdots \quad (9)$$

[Expression 10]

$$E_{23} = \sum_{t=1}^{T-1} |Y'(t)| + \sum_{t=1}^{T-1} |Z'(t)| - \sum_{t=1}^{T-1} |X'(t)| \quad \cdots \quad (10)$$

In a case where the maximum value of the evaluation function $E_{21}$ is the largest in the maximum values of the evaluation functions $E_{21}$ to $E_{23}$, the specification unit 14 specifies that X'(t) and Y'(t) are the motion data in the representative direction. In this case, the determination unit 15 determines a conversion parameter of which the evaluation function $E_{21}$ is equal to or more than the second standard (for example, maximum) as the parameter used for conversion.

In a case where the maximum value of the evaluation function $E_{22}$ is the largest in the maximum values of the evaluation functions $E_{21}$ to $E_{23}$, the specification unit 14 specifies that X'(t) and Z'(t) are the motion data in the representative direction. In this case, the determination unit 15 determines a conversion parameter of which the evaluation function $E_{22}$ is equal to or more than the second standard (for example, maximum) as the parameter used for conversion.

In a case where the maximum value of the evaluation function $E_{23}$ is the largest in the maximum values of the evaluation functions $E_{21}$ to $E_{23}$, the specification unit 14 specifies that Y'(t) and Z'(t) are the motion data in the representative direction. In this case, the determination unit 15 determines a conversion parameter of which the evaluation function $E_{23}$ is equal to or more than the second standard (for example, maximum) as the parameter used for conversion.

Furthermore, for example, in a case where the mixing degree is three, the specification unit 14 calculates an

evaluation value $E_3$ indicated in the following expression (11) regarding each of the plurality of conversion parameters.
[Expression 11]

$$E_3 = \sum_{t=1}^{T-1} |X'(t)| + \sum_{t=1}^{T-1} |Y'(t)| + \sum_{t=1}^{T-1} |Z'(t)| \quad \cdots \quad (11)$$

In a case where the mixing degree is three, the specification unit 14 specifies that X'(t), Y'(t), and Z'(t) are the motion data in the representative direction. In this case, the determination unit 15 determines a conversion parameter of which the evaluation function $E_3$ is equal to or more than the second standard (for example, maximum) as the parameter used for conversion.

In the above example, the determination unit 15 has converted the parameter for each motion section extracted by the extraction unit 12. The determination unit 15 may estimate the conversion parameter for each type of the motion. For example, the determination unit 15 may determine a parameter of which a second motion feature amount in a specified motion direction is equal to or more than the second standard for each period (section) determined based on the type of the motion. The type of the motion is, for example, walking or direction change, and it is assumed that a motion pattern or a motion detection algorithm for each type of the motion, or parameter of the same be stored in the first storage unit 10 in advance. Then, the determination unit 15 may determine the type of the motion based on matching with the stored motion pattern or may determine the type of the motion by using the motion detection algorithm.

Furthermore, the determination unit 15 may determine the conversion parameter for each motion having a common mixing degree or a similar mixing degree. The determination unit 15 may determine the parameter of which the motion feature amount in the specified motion direction is equal to or more than the second standard for each period (section) determined based on the mixing degree.

In a case where the conversion parameter is determined for each type of the motion or each motion having a common mixing degree or a similar mixing degree, there is a possibility that the target motion data includes the plurality of motion sections. In that case, for example, the determination unit 15 may determine a parameter of which a value obtained by adding or averaging the evaluation functions in the plurality of sections is equal to or more than a predetermined standard (for example, maximum) as the parameter used for conversion.

Furthermore, in a case where the representative direction has been set in advance, the determination unit 15 may determine the conversion parameter of which the motion feature amount in the set representative direction is equal to or more than the second standard (for example, maximum). For example, in a case where the motion is straight walking, it can be estimated that the representative direction be the rotation direction around the frontal-horizontal axis. Therefore, the representative direction is set to be the rotation direction around the frontal-horizontal axis in advance. In a case where the representative direction has been set in advance, the determination unit 15 may determine, for example, a conversion parameter of which the motion feature amount in the set representative direction is equal to or more than the predetermined standard (for example, maximum).

FIG. 9 is a diagram illustrating an example of motion data before and after the motion conversion according to the number of representative directions. In FIG. 9, a solid line indicates the motion data after conversion X'(t), Y'(t), and Z'(t), and a dotted line indicates the motion data before conversion X(t), Y(t), and Z(t).

FIG. 9(a) illustrates an example of motion conversion in a case where the representative direction is a single direction and X'(t) is the representative direction. In the example illustrated in FIG. 9(a), X(t) is converted to be increased, and Y(t) and Z(t) are converted to be decreased.

FIG. 9(b) illustrates an example of motion conversion in a case where the representative direction includes two directions and X'(t) and Y'(t) are the representative directions. In the example illustrated in FIG. 9(b), X(t) and Y(t) are converted to be increased, and Z(t) is converted to be decreased.

FIG. 9(c) illustrates an example of motion conversion in a case where the representative direction includes three directions and X'(t), Y'(t), and Z'(t) are the representative directions. In the example illustrated in FIG. 9(c), X(t), Y(t), and Z(t) are converted to be increased.

Next, the conversion unit 16 converts the motion data by using the conversion parameter determined by the determination unit 15 (step S107). For example, the conversion unit 16 substitutes the conversion parameter $\theta = (\alpha, \beta, \gamma)$ determined by the determination unit 15 into each of the expressions (2) to (4). Then, X'(t), Y'(t), and Z'(t) to which the conversion parameter has been substituted are the converted motion data.

In a case where the conversion parameter is known, the conversion unit 16 may convert the motion data by using the known conversion parameter. Alternatively, the conversion unit 16 may compare the known conversion parameter with the determined conversion parameter and select either one of the conversion parameters.

For example, in a case where a difference of the attachment position of the sensor 4 has been known, the conversion unit 16 may select a conversion parameter from among the conversion parameters calculated based on the attachment position in the past. Alternatively, in a case where a motion based on a habit of a wearer and an abnormal motion have been known, the conversion unit 16 may select a conversion parameter from among the conversion parameters calculated in the past based on the expected attachment position of the sensor 4.

As illustrated in FIG. 6, in a case where the processing in steps S104 to S107 has been completed regarding all the motion sections, the information processing apparatus 1 terminates the repetitive processing (step S108).

FIG. 10 is a diagram illustrating an example of the motion data before and after the motion conversion. FIGS. 10(a) to 10(c) illustrate motion data in the representative direction of the motion data in three directions including the single representative direction. FIG. 10(a) is motion data in a case where the sensor 4 is attached at the normal position. FIG. 10(b) is motion data in a case where the sensor 4 is attached to a position that is not normal (rotationally shifted position). FIG. 10(c) is motion data obtained by converting the motion data in FIG. 10(b).

In the motion data illustrated in FIG. 10(b), the sensor 4 is not normally attached. Therefore, the motion amount in the motion data in the representative direction is smaller than the motion amount in the motion data in FIG. 10(a). On the other hand, the data illustrated in FIG. 10(c) has a larger motion amount than the motion amount in the motion data illustrated in FIG. 10(b) and is close to the motion data in FIG. 10(a). This is because, in the data illustrated in FIG. 10(c), conversion is performed by using the conversion parameter of which the motion feature amount in the representative direction after conversion is maximized.

FIG. 11 is a diagram illustrating a comparative example of motion data before and after the motion conversion. FIG. 11(a) illustrates a part of the motion data in the representative direction. FIG. 11(a) illustrates the motion data in the representative direction in a case where the sensor 4 is attached at the normal position and the motion data in the representative direction before and after the conversion in a case where the sensor 4 is attached to the position that is not normal (rotationally shifted position).

As illustrated in FIG. 11(a), the motion data in the representative direction after the conversion is close to the motion data in the representative direction in a case where the sensor 4 is attached at the normal position.

FIG. 11(b) illustrates a part of motion data in a direction other than the representative direction. FIG. 11(b) illustrates the motion data in the direction other than the representative direction in a case where the sensor 4 is attached at the normal position and the motion data in the direction other than the representative direction before and after the conversion in a case where the sensor 4 is attached to the position that is not normal (rotationally shifted position).

As illustrated in the example in FIG. 11(b), regarding the motion data in the direction other than the representative direction, a motion amount in a case where the sensor 4 is attached at the position that is not normal may be larger than a motion amount in a case where the sensor 4 is attached at the normal position. On the other hand, the motion data after the conversion is close to the motion data in a case where the sensor 4 is attached at the normal position.

Next, the calculation unit 17 calculates a feature amount by using the converted motion data (step S109). The calculation unit 17 may specify the type of the motion (for example, walking, direction change, and the like) by using the converted motion data. The calculation unit 17 may, for example, specify the type of the motion by comparing the converted motion data and a pattern of each type of the motion which has been stored in advance. In a case where the type of the motion has been already set, the calculation unit 17 may use the type of the motion for quantification.

Furthermore, for example, in a case where the type of the motion is walking, the calculation unit 17 calculates (quantifies) the feature amount such as a walking speed and a stride length. Furthermore, for example, in a case where the type of the motion is direction change, the calculation unit 17 calculates the feature amount such as a rotation direction and a rotation amount (angle).

As described above, the information processing apparatus 1 according to the present embodiment determines the conversion parameter of which the motion feature amount in the representative direction after the conversion becomes equal to or more than the predetermined standard (for example, maximum) and converts the motion data. Then, as illustrated in FIGS. 10 and 11, the motion feature amount in the representative direction after the conversion is increased so that motion data close to the motion data in a case where the sensor 4 is attached at the normal position can be obtained.

Therefore, even in a case where an initial attachment position of the sensor is different from the expected position and in a case where the attachment position is moved to a position different from the expected position by a movement of the person to be measured, deterioration in accuracy of the quantification of the motion can be suppressed.

Furthermore, for example, by accumulating and visualizing the conversion parameter, the information processing apparatus 1 can recognize a change of a state of the sensor 4 (attachment position) and abnormalities. The determined conversion parameter can be used to determine a motion direction of a motion assisting device such as a powered suit.

In addition, for example, it is not easy to install a large monitoring device used in a hospital to recognize a state of a patient (person to be measured) in a home of the patient or a nursing care facility from viewpoint of securing installation space, introduction cost, requirements for operation skills, and the like.

Therefore, it is desirable to recognize the state of the person to be measured such as a patient by an inexpensive device which can be easily used. By using the information processing apparatus 1 according to the present embodiment, since the state of the person to be measured can be recognized by using, for example, a small device such as a gyro sensor, the motion of the person to be measured can be inexpensively and easily quantified.

<Example of Information Processing Apparatus According to Second Embodiment>

An information processing apparatus according to a second embodiment will be described with reference to the drawings. Hereinafter, it is assumed that configurations and functions that are not specifically described be similar to those in the information processing apparatus according to the first embodiment.

FIG. 12 is a diagram illustrating an example of the information processing apparatus according to the second embodiment. In addition to the configuration included in the information processing apparatus 1 according to the first embodiment, the information processing apparatus 1 according to the second embodiment includes a first abnormality determination unit 18 and a second abnormality determination unit 19.

The first abnormality determination unit 18 determines a state abnormality of a sensor 4 based on variation in motion data.

The second abnormality determination unit 19 determines the state abnormality of the sensor 4 based on a change according to a lapse of time of a mixing degree.

<Example of Processing Flow in Second Embodiment>

FIGS. 13 and 14 are flowchart illustrating an example of a flow of the information processing apparatus according to the second embodiment. The flowcharts illustrated in FIGS. 13 and 14 include steps S204 to S207, in addition to the processing included in the flowchart in the first embodiment illustrated in FIG. 6. Steps S201 to S203 in FIG. 13 and steps S208 to S213 in FIG. 14 are similar to the processing in steps S101 to S109 in FIG. 6.

A first communication unit 11 receives motion data indicating a motion measured by the sensor 4 (step S201). An extraction unit 12 extracts data in a section indicating a motion to be measured from the received motion data (step S202). The information processing apparatus 1 starts repetitive processing for each motion section extracted in step S202 (step S203). In step S203, when it is assumed that a reference N indicates the number of motion sections, the repetitive processing is performed in each motion section i (i = one to N).

The first abnormality determination unit 18 determines whether variation in motion data in a representative direction in the motion section i is equal to or more than a threshold (first abnormality determination threshold) (step S204). For example, the first abnormality determination unit 18 determines whether or not a maximum value of a motion amount in the representative direction exceeds the first abnormality determination threshold, for example, in a target motion section. Alternatively, the first abnormality determination unit 18 may calculate a difference between a statistic value (maximum value, average value, and the like) of a motion amount of a certain motion section and a statistic value of a motion amount of a preceding motion section and determine whether the difference is equal to or more than the first abnormality determination threshold.

In a case where the variation is equal to or more than the first abnormality determination threshold (YES in step S204), the first abnormality determination unit 18 determines that the state of the sensor 4 is abnormal (step S207). Then, the information processing apparatus 1 terminates the processing.

In a case where the variation in the motion data is large, for example, it is assumed that the sensor 4 vibrate along with the motion of the person to be measured by loosely attaching the sensor 4 and not fixing the sensor 4. In that case, since it is difficult to accurately quantify the motion even when the motion data is converted, in a case where it has been determined that the state of the sensor 4 is abnormal, the information processing apparatus 1 terminates the processing.

FIG. 15 illustrates examples of motion data at the time of walking in a case where the sensor is fixed and in a case where the sensor is not fixed. FIG. 15(a) illustrates the motion data at the time of walking in a case where the sensor is fixed. FIG. 15(b) illustrates the motion data at the time of walking in a case where the sensor is not fixed.

For example, a motion amount indicated by a broken line portion in the motion data illustrated in FIG. 15(b) is larger than a motion amount in the motion data illustrated in FIG. 15(a). In a case where the abnormal motion data is included as illustrated in FIG. 15(b), the first abnormality determination unit 18 determines that the state of the sensor is abnormal.

As illustrated in FIG. 13, the second abnormality determination unit 19 calculates a change amount according to a lapse of time of the mixing degree in the extracted motion section (step S205). For example, the second abnormality determination unit 19 repeats processing for calculating the mixing degree regarding a cumulative value of motion data in a predetermined time width in the motion section i and calculating a mixing degree by changing (for example, increase) the time width and the cumulative value so as to calculate a change amount according to the lapse of time of the mixing degree.

Then, the second abnormality determination unit 19 determines whether or not the type of the motion changes in a single motion section (step S206). In step S206, for example, in a case where the mixing degree changes in the single motion section, the second abnormality determination unit 19 determines that the type of the motion changes.

In a case where it is determined that the type of the motion changes in the single motion section (YES in step S206), the second abnormality determination unit 19 determines that the state of the sensor 4 is abnormal (step S207). Then, the information processing apparatus 1 terminates the processing.

For example, in a case where a difference between a conversion parameter in a certain motion section and a conversion parameter in a motion section separated from the certain motion section by a predetermined time is equal to or more than a threshold (second abnormality determination threshold), the second abnormality determination unit 19 may determine that the state of the sensor 4 is abnormal.

For example, in a case where the conversion parameter in a specific motion section is compared with a known conversion parameter and a difference is equal to or more than the second abnormality determination threshold, the second abnormality determination unit 19 may determine that the state of the sensor 4 is abnormal.

FIG. 16 is a diagram illustrating motion data of a leg in a case where an attachment position gradually changes. FIG. 16(a) is the motion data of the leg in the frontal-horizontal axis direction in a case where the attachment position gradually changes. In the example illustrated in FIG. 16(a), a motion amount gradually decreases. FIG. 16(b) is the motion data of the leg in the sagittal-horizontal axis direction in a case where the attachment position gradually changes. In the example illustrated in FIG. 16(b), a motion amount gradually increases.

As illustrated in FIG. 16, for example, in a case where the motion amount in the frontal-horizontal axis direction that is the representative direction decreases at the time of walking and the motion amount in the other direction increases, it is assumed that the sensor 4 be gradually moved from the normal attachment position.

FIG. 17 is a diagram illustrating a change in a mixing degree in a case where the attachment position gradually changes. As illustrated in FIG. 17, since a cumulative contribution ratio exceeds a cumulative contribution ratio threshold regarding each axis at the beginning of the motion section, the mixing degree is one. However, as time elapses, the cumulative contribution ratio of the single axis decreases and falls below the cumulative contribution ratio threshold. Therefore, the mixing degree changes from one to two.

In the examples illustrated in FIGS. 16 and 17, the second abnormality determination unit 19 determines that the type of the motion changes in the single motion section and determines that the state of the sensor 4 is abnormal.

In a case where it has been determined that the type of the motion has not changed in step S206, the mixing degree determination unit 13 determines a mixing degree indicating the number of representative directions in the motion section i (step S208). The specification unit 14 specifies a representative direction in which the motion feature amount is the first standard (for example, maximum) based on the mixing degree (step S209). The determination unit 15 determines a parameter (conversion parameter) used for the conversion of the motion data so that the motion feature amount in the representative direction is equal to or more than the second standard (for example, maximum) (step S210).

Next, the conversion unit 16 converts the motion data by using the conversion parameter determined by the determination unit 15 (step S211). In a case where the processing in steps S204 to S211 has been completed regarding all the motion sections, the information processing apparatus 1 terminates the repetitive processing (step S212). Then, the calculation unit 17 calculates a feature amount by using the converted motion data (step S213).

As described above, the information processing apparatus 1 according to the present embodiment determines whether or not the state of the sensor 4 is abnormal, and in a case where the state of the sensor 4 is abnormal, the information processing apparatus 1 terminates the processing. Therefore, the information processing apparatus 1 does not convert the abnormal motion data. Therefore, it is possible to avoid the quantification based on the abnormal motion data.

<Example of Information Processing Apparatus According to Third Embodiment>

An information processing apparatus according to a third embodiment will be described with reference to the drawings. Regarding the information processing apparatus according to the third embodiment, it is assumed that configurations and functions that are not specifically described be similar to those in the information processing apparatus according to the first embodiment or the second embodiment. Since the configuration of the information processing apparatus according to the third embodiment is similar to the configuration of the information processing apparatus according to the first embodiment or the second embodiment, the description thereof will be omitted.

FIG. 18 is a diagram illustrating an example of motion data in a case where a motion for walking backward during walking is included. FIG. 18 illustrates motion data in a representative direction if motion data of left and right legs. As illustrated in FIG. 18, for example, in a case where forward walking motion data partially includes backward walking motion data, positive and negative signs of the motion data are reversed. In a case where the motion data illustrated in FIG. 18 is converted, there is a possibility that the conversion result is not normal.

Therefore, in a case where the motion data has been preset to be walking, a determination unit 15 determines a conversion parameter based on a motion feature amount independent of whether the motion data is positive or negative. For example, the determination unit 15 determines the conversion parameter so that the motion feature amount independent of whether the data is positive or negative in the representative direction is equal to or more than a second standard (for example, maximum). The motion feature amount independent of whether the data is positive or negative is a motion feature amount having a positive value, for example, an absolute value of a peak of the motion data and an area of a peak section.

As described in the first embodiment, the determination unit 15 may determine the conversion parameter so that the evaluation function in any one of the expressions (5) to (11) is equal to or more than a predetermined standard (for example, maximum). Since the expressions (5) to (11) are evaluation functions based on absolute values of each motion data X'(t), Y'(t), and Z'(t), the data is positive regardless of the positive or negative of the motion data.

During walking, the legs rotate in the rotation direction around the frontal-horizontal axis as the representative direction regardless of a traveling direction. Therefore, by using the conversion parameter calculated based on the motion feature amount independent of whether the motion data is positive or negative, a conversion unit 16 can obtain appropriate converted data even in a case where the motion data of forward walking and the motion data of backward walking are mixed.

FIG. 19 is a diagram illustrating motion data before and after conversion in a case where a conversion parameter is a local solution. The reference $\tau$ indicates a section number assigned for each motion section. In an example illustrated in FIG. 19, a conversion parameter in the section $\tau$ is $\theta$ = (150, 60, 240), and a conversion parameter in a section $\tau$ + 1 is $\theta$ = (150, 240, 60). That is, it is assumed that conversion parameters in two directions other than the representative direction are reversed.

However, usually, the state of the sensor does not largely change in a short time. Therefore, there is a high possibility that the conversion parameters illustrated in FIG. 19 are not appropriate. Therefore, the determination unit 15 does not adopt the conversion parameter which largely changes in a short time.

For example, the determination unit 15 calculates a conversion parameter by using an evaluation function to which a constraint is added as in the following expression (12).

[Expression 12]

$$E_{\tau+1} = \sum_{t=1}^{T-1} |X'(t)| - \sum_{t=1}^{T-1} |Y'(t)| - \sum_{t=1}^{T-1} |Z'(t)| - \epsilon \|\theta_{\tau+1} - \theta_{\tau}\| \quad \cdots (12)$$

The expression (12) is an example in which a constraint is added to the expression (5). The reference $\theta_{\tau+1}$ indicates a conversion parameter in a first motion section, and the reference $\theta_{\tau}$ indicates a conversion parameter, which is calculated in advance, in a single or a plurality of second motion sections that is a predetermined time before the first motion section. The reference $\varepsilon$ is a parameter indicating a degree of allowance regarding a difference between a conversion parameter in a target motion section and a conversion parameter in a preceding motion section.

As indicated in the expression (12), the determination unit 15 subtracts a value obtained by multiplying a difference (for example, Euclidean distance) between the conversion parameter in the first motion section ($\tau$ + 1) and the conversion parameter in the second motion section ($\tau$), which is calculated in advance, that is a predetermined time before the first motion section with a predetermined parameter.

For example, the determination unit 15 substitutes a plurality of conversion parameter candidates into $\theta_{\tau+1}$ and determines a conversion parameter so that the evaluation function indicated in the expression (12) is maximized. By adding the constraint, the evaluation function decreases according to whether the difference between the conversion parameter in the first motion section ($\tau$ + 1) and the conversion parameter in the second motion section ($\tau$), which is calculated in advance, before a predetermined time (for example, Euclidean distance) increases. Therefore, a possibility that the conversion parameter in a case where the difference (for example, Euclidean distance) is large is used for the conversion is low. That is, in a case where the conversion parameter largely changes in a short time, there is a low possibility that the conversion parameter is used for conversion.

The expression (12) indicates the evaluation function corresponding to the expression (5). In addition, the determination unit 15 may use the expressions (6) to (11) to determine the conversion parameter by adding a similar constraint (fourth term in expression (12)).

The second motion section is, for example, a motion section previous to the first motion section. The second motion section may be, for example, a motion section after the first motion section.

For example, the determination unit 15 may apply the Kalman filter and a state space model and use the motion data and the conversion parameter in the motion data previous to the target motion section to estimate a conversion parameter of which a change amount in a short time is small.

As described above, the determination unit 15 calculates a difference between a conversion parameter candidate in the first motion section in the motion data and the determined conversion parameter in the second motion section which is a predetermined time before the first motion section. Then, the determination unit 15 determines the conversion parameter in the first motion section from among the conversion parameter candidates based on the difference. Therefore, for example, the information processing apparatus 1 can determine an appropriate conversion parameter even when the obtained motion data includes abnormal motion data.

FIG. 20 is a diagram illustrating section separation of the motion data including the abnormal motion. As illustrated in FIG. 20, for example, there is a case where tendency in the motion data largely changes in a short time. The reason why the tendency in the motion data largely changes in a short time is because the state of the sensor 4 (for example, attachment position) is changed due to an impact caused when, for example, the person to be measured touches the sensor 4 by hands or the sensor 4 collides with an obstacle. In a case where the motion data having different sensor states is included in the single motion section, thereafter, when the determination of the mixing degree and the parameter conversion are performed for each motion section, there is a possibility that an appropriate result cannot be obtained.

In a case where an impact is applied to the sensor 4 when the person to be measured touches the sensor 4 by hands or the sensor 4 collides with an obstacle, abnormal motion data based on the impact may occur. Therefore, in a case where the motion data in the certain motion section includes the abnormal motion data, an extraction unit 12 separates the motion section into two sections before and after the abnormal motion data. For example, in a case where a peak value of the motion data is equal to or more than an extraction threshold, the extraction unit 12 determines that the motion data is the abnormal motion data. Then, the extraction unit 12 outputs the separated motion section to a mixing degree determination unit 13.

As described above, the extraction unit 12 detects a portion indicating an abnormal motion in the motion data, separates a section before the portion indicating the abnormal motion from a section after the portion indicating the abnormal motion, and extracts each separated section. By separating the section, it is possible to prevent that the motion data based on the plurality of sensor states is included in the single motion section. Then, the determination unit 15 can determine an appropriate parameter by determining the conversion parameter for each of the separated motion sections.

FIG. 21 is a diagram illustrating an example of motion data of a direction change after the conversion in the plurality of motion sections. A motion section (1) and a motion section (2) in FIG. 21 are motion sections at the time of direction change. It is assumed that a representative direction of the direction change be X'(t) and Y'(t). As illustrated in FIG. 21, although a motion amount of Y'(t) is the largest in the motion section (1), a motion amount of X'(t) is the largest in the motion section (2).

As illustrated in FIG. 21, even in the motion data of the same type of the motion, a direction, in which the motion amount is the largest, of the representative directions (referred to as dominant axial direction below) may different for each motion section. In a case where the motion data is converted and quantified by using the motion data, accuracy may be deteriorated. Therefore, in a case where a specification unit 14 specifies two or more representative directions, it is preferable to distinguish the dominant axial direction.

For example, in a case where the representative direction includes two or more directions, the determination unit 15 calculates a motion amount of the motion data before the conversion in each representative direction and determines a direction in which the motion amount is the largest (dominant axial direction). Then, the determination unit 15 determines a conversion parameter of which a motion feature amount after the conversion in the dominant axial direction is maximized. For example, in a case where the dominant axial direction of the motion data before the conversion is Y(t), the determination unit 15 determines a conversion parameter of which a motion feature amount based on Y(t) (for example, second term in expression (8)) is maximized.

However, in a case where the type of the motion, such as walking, is known in advance, the determination unit 15 may determine the dominant axial direction according to the type of the motion. For example, in a case where the type of the motion is walking, the rotation direction around the frontal-horizontal axis may be determined as the dominant axial direction. In a case where the same type of the motion is repeated a plurality of times, the determination unit 15 may determine a direction in which a total motion amount is the largest in the motion data corresponding to the plurality of times of motions or a direction in which the total motion amount is maximized after conversion as the dominant axial direction.

As described above, in a case where there are two representative directions, since the determination unit 15 determines a parameter of which the motion feature amount in the direction, in which the motion amount is maximized in the representative directions, is the largest, deterioration in the quantification accuracy can be suppressed.

FIG. 22 is a diagram illustrating motion data after the conversion of the direction change. FIG. 22(a) illustrates motion data in a case where the conversion has been normally performed. FIG. 22(b) illustrates motion data in a case where the conversion has not been normally performed.

In the present embodiment, a case where Y'(t) is positive indicates rightward turning, and a case where Y'(t) is negative indicates leftward turning. However, as illustrated in FIG. 22(b), there is a case where the motion data which originally indicates rightward turning is converted into negative data.

In the example illustrated in FIG. 22, it is assumed that X'(t) be motion data in the rotation direction around the frontal-horizontal axis. In a case where the person turns during forward walking, the legs rotate forward regardless of the turning direction. Therefore, X'(t) normally indicates a positive value. However, in FIG. 22(b), since X'(t) is negative data, it is found that the motion data is abnormal. That is, for example, if it is found that the motion data is the motion data at the time of forward walking, it is possible to determine whether the converted motion data is normal based on the positive or negative of the motion data of X'(t).

Therefore, for example, in a case where the type of the motion is the direction change, the determination unit 15 determines a conversion parameter so that X'(t) is positive motion data. In the following expression (13), an absolute value of X'(t) is removed from the expression (8).

[Expression 13]

$$E'_{21} = \sum_{t=1}^{T-1} X'(t) + \sum_{t=1}^{T-1} |Y'(t)| - \sum_{t=1}^{T-1} |Z'(t)| \quad \cdots (13)$$

As indicated by the expression (13), by removing the absolute value of the motion data X'(t) that is not the rotation direction of the dominant axis of the motion data of the plurality of representative directions, the conversion parameter in a case where X'(t) is negative is hardly adopted as a parameter actually used for conversion. Therefore, the conversion parameter is determined so that the motion data of X'(t) is positive.

As described above, if a single direction of the plurality of representative directions is known, the information processing apparatus 1 determines the conversion parameter based on the positive or negative of the motion data in the known direction. Therefore, a possibility that the positive and negative of the original motion data is reversed can be suppressed. FIG. 23 is a diagram illustrating the rotation direction of the leg. Usually, in a case where the motion is straight walking, the rotation direction around the frontal-horizontal axis is the representative direction. The specification unit 14 specifies the representative direction in the processing in step S105 or S209. However, there is a case where motion data in the direction other than the representative direction is switched and estimated by the conversion processing of the motion data.

For example, in a case where the rotation direction around the frontal-horizontal axis is X'(t), there is a case where the motion data in the rotation direction around the sagittal-horizontal axis is output as Y'(t) or Z'(t). In addition, there is a case where the motion data in the rotation direction around the vertical axis is output as Z'(t) or Y'(t).

Usually, in a case where the motion is straight walking, the motion amount in the rotation direction around the sagittal-horizontal axis is smaller than the motion amount in the rotation direction around the vertical axis. This is because, although there is a possibility that a motion for rotating an ankle is performed during walking, a motion for laterally swinging the ankle is not usually performed. Therefore, the determination unit 15 determines a conversion parameter so that the motion data in the direction, in which the motion amount is large, of the motion data in the direction which is not the representative direction is the motion data in the rotation direction around the vertical axis (in the present embodiment, Y'(t)).

For example, the determination unit 15 may calculate the conversion parameter based on the following expression (14) obtained by adding a constraint condition to the expression (5). In a case where the expression (14) is used, it is assumed that the motion data in the rotation direction around the vertical axis be Y'(t) and that the rotation direction around the sagittal-horizontal axis be Z'(t).

[Expression 14]

$$E'_{11} = \sum_{t=1}^{T-1} |X'(t)| - \sum_{t=1}^{T-1} |Y'(t)| - \sum_{t=1}^{T-1} |Z'(t)|$$

$$s.t. \, |Y'(t)| > |Z'(t)| \quad \cdots (14)$$

An example of the case of straight walking has been described above. However, the present invention is not limited to the case of straight walking. If a magnitude relation of the motion data in the direction other than the representative direction is known, it is preferable that the determination unit 15 determine the conversion parameter by using the magnitude relation as a constraint.

As described above, in a case where the magnitude relation of the motion data in the direction other than the representative direction is known, the information processing apparatus 1 determines the conversion parameter by using the magnitude relation as the constraint. Therefore, it is possible to prevent that the motion data in the direction other than the representative direction is switched by the conversion.

<First Example>

As a first example, an example will be described in which the characteristics of walking during straight walking is quantified in a case where a person to be measured wears a single sensor 4 on a left leg and five sensors 4 on a right leg.

FIG. 24 is motion data from each sensor 4 obtained by an extraction unit 12 in the first example. In FIG. 24, only the motion data in a representative direction is illustrated, and illustration of the motion data in a direction other than the representative direction is omitted. In the example illustrated in FIG. 24, since the sensors 4 attached on the right leg are attached at different positions, pieces of motion data are different from each other.

FIG. 25 is a diagram illustrating an example of a motion extraction algorithm. The extraction unit 12 obtains an algorithm for extracting a previously designated motion from a motion extraction algorithm DB 10a and extracts data in a section indicating a motion to be detected.

As illustrated in FIG. 25, the motion extraction algorithm includes an algorithm number and each parameter used for extracting the motion. The parameters are, for example, a peak value of a composite value, a peak interval of the composite value, a peak width of the composite value, and a peak gradient of the composite value. Each algorithm includes, for example, whether or not the parameter is required (O or X) and a threshold of each parameter. The motion extraction algorithm may include an extraction method using each parameter.

The motion extraction algorithm is stored, for example, for each type of the motion. In the present example, the motion extraction algorithm DB 10a applies an algorithm No. 1 corresponding to a walking motion.

FIG. 26 is a diagram illustrating an example of an extracted motion section. As illustrated in FIG. 26, in the motion data, a portion indicating the motion to be detected is extracted.

Next, a mixing degree determination unit 13 obtains a determination index corresponding to a target motion from a determination index DB 10b including a determination index of a mixing degree and determines the mixing degree based on the determination index.

FIG. 27 is a diagram illustrating an example of the determination index of the mixing degree. As illustrated in FIG. 27, the determination index DB 10b stores a definition number of the determination index, the target motion, and the determination index of the mixing degree in association with each other. Furthermore, the determination index of the mixing degree includes a threshold of a cumulative contribution ratio in main component analysis, a threshold of a ratio of independent component analysis, a threshold of a value indicating variation in other motion data (for example, covariance and standard deviation). That is, the determination index of the mixing degree is a determination threshold used for evaluating variation in the motion data including three-dimensional motion directions (for example, minimum threshold of magnitude of variation).

The mixing degree determination unit 13 uses the minimum number of axes of the number of axes of which a value corresponding to the determination index of the motion data is equal to or more than a threshold as the mixing degree. In the present example, since the target motion is straight walking, the determination index of the definition No. 1 is used for determination of the mixing degree. The mixing degree determination unit 13 determines the mixing degree by using the determination index of the definition No. 1 and assumes the mixing degree as "one".

Next, a specification unit 14 specifies the representative direction based on the mixing degree. In the present example, since the mixing degree is "one", the specification unit 14 specifies that a direction in which a motion feature amount is the largest in motion feature amounts in a plurality of directions as the representative direction.

Next, the determination unit 15 determines the conversion parameter so that the motion feature amount in the representative direction is maximized.

FIG. 28 is a diagram illustrating an example of information stored in a state conversion amount DB. The determination unit 15 makes the state conversion amount DB 10c store the determined conversion parameter. As illustrated in FIG. 28, the state conversion amount DB stores a date and time, a motion start time, a motion end time, and a state conversion amount in association with each other. The state conversion amount includes, for example, an X-axis rotation amount (a), a Y-axis rotation amount ($\beta$), and a Z-axis rotation amount ($\gamma$). The state conversion amount corresponds to the conversion parameter.

The conversion parameter determined by the determination unit 15 is stored in the state conversion amount DB 10c and a certain amount of data is accumulated and visualized so that a change in a sensor state (position) and abnormalities can be recognized.

Next, a conversion unit 16 converts the motion data by using the conversion parameter determined by the determination unit 15. FIG. 29 is a diagram illustrating an example of motion data before and after the conversion. In FIG. 29, X'(t) is motion data in a rotation direction around a frontal-horizontal axis, Y'(t) is motion data in a rotation direction around a vertical axis, and Z'(t) is motion data in a rotation direction around a sagittal-horizontal axis. Furthermore, FIG. 29 includes motion data before after conversion in three directions (X'(t), Y'(t), and Z'(t)) of a right leg (1) and motion data before and after conversion of X'(t) of the right leg (2) to (5). Illustration of motion data of a left leg and motion data of Y'(t) and Z'(t) of the right leg (2) to (5) is omitted.

In FIG. 29, it is assumed that a conversion parameter of the right leg (1) be θ = (150, 60, 240), a conversion parameter of the right leg (2) be θ = (300, 330, 60), and a conversion parameter of the right leg (3) be θ = (180, 60, 360). In addition, it is assumed that a conversion parameter of the right leg (4) be θ = (330, 60, 240) and a conversion parameter of the right leg (5) be θ = (330, 360, 30).

As illustrated in FIG. 29, regarding X'(t), a motion amount is increased by the conversion. In addition, regarding Y'(t) and Z'(t), the motion amount is reduced by the conversion. In the present example, the motion is walking, and X'(t) is the representative direction. Therefore, the motion amount in the representative direction is increased by the conversion, and the motion amount in a direction other than the representative direction is reduced. The converted motion data is close to motion data in a case where the sensor 4 is attached at a normal position.

Next, the calculation unit 17 specifies the type of the motion by using the converted motion data and quantifies the motion. In the present example, the calculation unit 17 specifies that the converted motion data is walking. The calculation unit 17 quantifies the feature amount regarding walking based on the converted motion data and stores the feature amount in a feature amount DB 10d.

FIG. 30 is a diagram illustrating an example of the motion feature amount regarding walking stored in the feature amount DB. As illustrated in FIG. 30, the motion feature amount regarding walking indicates a date and time, a left leg walking speed, a right leg walking speed, a left leg stride length, and a right leg stride length.

The calculation unit 17 makes the feature amount DB 10d store, for example, an amplitude of the motion data as the walking speed. For example, the calculation unit 17 calculates a stride length based on an integration value of the motion data and makes the feature amount DB 10d store the stride length.

<Second Example>

As a second example, an example will be described in which the characteristics of walking during a direction change is quantified in a case where a person to be measured wears a single sensor 4 on a left leg and five sensors 4 on a right leg. In the present example, it is assumed that processing that is not specifically described be similar to the first example.

FIG. 31 is motion data from each sensor 4 obtained by an extraction unit 12 in the second example. In FIG. 31, only motion data in a representative direction is illustrated, and illustration of motion data in a direction other than the representative direction is omitted. In the example illustrated in FIG. 31, since the sensors 4 attached on the right leg are attached at different positions, pieces motion data are different from each other.

The extraction unit 12 obtains an algorithm for extracting a previously designated motion from a motion extraction algorithm DB 10a and extracts data in a section indicating a motion to be detected. In the present example, the data in the section indicating the motion to be detected is extracted by using an algorithm corresponding to the algorithm No. 2 in FIG. 25.

Next, a mixing degree determination unit 13 obtains a determination index corresponding to a target motion from a determination index DB 10b including a determination index of a mixing degree and determines the mixing degree based on the determination index. In the present example, the mixing degree determination unit 13 determines the mixing degree by using a mixing degree determination index associated with a definition No. 2 in FIG. 27. In the present example, it is assumed that the mixing degree determination unit 13 determine that the mixing degree is "two".

Next, a specification unit 14 specifies the representative direction based on the mixing degree. In the present example, since the mixing degree is "two", the specification unit 14 specifies two directions in which total motion feature amount of two directions is maximized as the representative directions.

Next, the determination unit 15 determines the conversion parameter so that the motion feature amount in the representative direction is maximized.

Next, a conversion unit 16 converts the motion data by using the conversion parameter determined by the determination unit 15. FIG. 32 is a diagram illustrating an example of motion data before and after conversion. In FIG. 32, X'(t) is motion data in a rotation direction around a frontal-horizontal axis, Y'(t) is motion data in a rotation direction around a vertical axis, and Z'(t) is motion data in a rotation direction around a sagittal-horizontal axis. Furthermore, FIG. 32 includes motion data before and after conversion in three directions (X'(t), Y'(t), and Z'(t)) of a right leg (1) and motion data before and after conversion of X'(t) of the right leg (2) to (5). Illustration of motion data of a left leg and motion data of Y'(t) and Z'(t) of the right leg (2) to (5) is omitted.

In FIG. 32, it is assumed that a conversion parameter of the right leg (1) be θ = (150, 120, 360), a conversion parameter of the right leg (2) be θ = (150, 150, 360), and a conversion parameter of the right leg (3) be θ = (150, 210, 360). In addition, it is assumed that a conversion parameter of the right leg (4) be θ = (150, 240, 180) and a conversion parameter of the right leg (5) be θ = (150, 150, 180).

Next, the calculation unit 17 specifies the type of the motion by using the converted motion data and quantifies the motion. In the present example, it is assumed that the calculation unit 17 specify that the converted motion data is data of the direction change. The calculation unit 17 quantifies a feature amount regarding the direction change based on the converted motion data and makes a feature amount DB 10d store the feature amount.

FIG. 33 is a diagram illustrating an example of the motion feature amount regarding the direction change stored in the feature amount DB. As illustrated in FIG. 33, the motion feature amount regarding the direction change indicates a date and time, a left leg rotation direction, a right leg rotation direction, a left leg rotation amount, and a right leg rotation amount.

For example, the calculation unit 17 determines that the motion is to rotate in the right rotation direction if the motion amount of the motion data is positive and determines that the motion is to rotate in the left rotation direction if the motion amount of the motion data is negative and makes the feature amount DB 10d store the rotation direction. For example, the calculation unit 17 calculates a rotation amount (rotation angle) based on an integration value for one period of the motion data and makes the feature amount DB 10d store the rotation amount.

<Example of Hardware Configuration of Information Processing Apparatus>

Next, an example of a hardware configuration of an information processing apparatus will be described with reference to an example in FIG. 34. As illustrated in the example in FIG. 34, a processor 111, a Random Access Memory (RAM) 112, and a Read Only Memory (ROM) 113 are connected to a bus 100. An auxiliary storage device 114, a medium connection unit 115, and a communication interface 116 are connected to the bus 100.

The processor 111 executes a program developed in the RAM 112. As the program to be executed, an information processing program for executing the processing in the embodiment may be applied.

The ROM 113 is a nonvolatile storage device which stores the program developed in the RAM 112. The auxiliary storage device 114 is a storage device which stores various information, and for example, a hard disk drive, a semiconductor memory, and the like may be applied to the auxiliary storage device 114. The medium connection unit 115 is provided to be connectable to a portable recording medium 118.

As the portable recording medium 118, a portable memory, an optical disc (for example, Compact Disc (CD) and Digital Versatile Disc (DVD)), a semiconductor memory, and the like may be applied. The information processing program for executing the processing in the embodiments may be recorded in the portable recording medium 118.

The first storage unit 10 illustrated in FIGS. 4 and 12 may be implemented by the RAM 112, the auxiliary storage device 114, and the like. The first communication unit 11 illustrated in FIGS. 4 and 12 may be implemented by the communication interface 116.

The extraction unit 12, the mixing degree determination unit 13, the specification unit 14, the determination unit 15, the conversion unit, and the calculation unit illustrated in FIGS. 4 and 12 and the first abnormality determination unit 18 and the second abnormality determination unit 19 illustrated in FIG. 12 may be implemented by executing the applied information processing program by the processor 111.

All of the RAM 112, the ROM 113, the auxiliary storage device 114, and the portable recording medium 118 are examples of computer-readable tangible storage media. These tangible storage media are not temporary media such as a signal carrier.

<Others>

The present embodiment is not limited to the embodiment described above, and various configurations and embodiments can be made without departing from the scope of the present embodiment.

REFERENCE SIGNS LIST

1 information processing apparatus

2 network

3 relay device

4 sensor

10 first storage unit

11 first communication unit

12 extraction unit

13 mixing degree determination unit

14 specification unit

15 determination unit

16 conversion unit

17 calculation unit

18 first abnormality determination unit

19 second abnormality determination unit

111 processor

112 RAM

113 ROM

## Claims

**1.** An information processing apparatus (1) comprising:

a communication unit (11) configured to receive original motion data regarding a motion from a sensor (4) for measuring the motion;
an extraction unit (12) configured to extract, from the original motion data, motion data of one or more first motion sections in which a motion to be detected is indicated, calculate a value of a temporal change amount of the motion data as a first motion feature amount and extract one or more second motion sections in which the first motion feature amount is equal to or more than a first extraction threshold from the one or more first motion sections;
a mixing degree determination unit (13) configured to determine, for each of the one or more second motion sections, a mixing degree indicating a number of representative moving directions of the motion to be detected;
a specification unit (14) configured to determine, for each of the one or more second motion sections, a representative moving direction based on the mixing degree; and
a determination unit (15) configured to determine a parameter used for conversion of the motion data, of which a second motion feature amount, indicating a value of a temporal change amount of the motion data in the representative moving direction after conversion, is equal to or more than a second threshold.

**2.** An information processing system comprising:

a sensor (4) configured to measure a motion; and
an information processing apparatus according to claim 1.

**3.** The information processing system according to claim 2, wherein
the extraction unit (12) obtains an algorithm for extracting the motion data of one or more first motion sections and obtains, from the algorithm, an extraction threshold as the first threshold.

**4.** The information processing system according to claim 2 or 3, wherein
the determination unit (15) determines the parameter used for conversion of the motion data of which the second motion feature amount in the representative moving direction is maximized.

5. The information processing system according to any one of claims 2 to 4, wherein the extraction unit (12) uses a composite value of the motion data for each of the moving directions as the first motion feature amount.

6. The information processing system according to any one of claims 2 to 5, wherein the mixing degree determination unit (13) determines the mixing degree by using a main component analysis.

7. The information processing system according to any one of claims 2 to 6, wherein the information processing apparatus (1) includes a conversion unit that converts the motion data by using the determined parameter.

8. The information processing system according to any one of claims 2 to 7, wherein the determination unit (15) determines the parameter of which the second motion feature amount is equal to or more than the second threshold for each period determined based on the type of the motion.

9. The information processing system according to any one of claims 2 to 8, wherein the information processing apparatus (1) includes a first abnormality determination unit (18) that determines a state abnormality of the sensor based on variation in the motion data.

10. The information processing system according to any one of claims 2 to 9, wherein the determination unit (15) determines the parameter of which the second motion feature amount having a positive value is equal to or more than the second threshold in a case where the type of the motion is walking.

11. The information processing system according to any one of claims 2 to 10, wherein the determination unit (15) determines the parameter in a first motion section from among parameter candidates based on a difference between the parameter candidate in the first motion section of the motion data and a parameter determined for a single or a plurality of second motion sections that is a predetermined time before or after the first motion section.

12. The information processing system according to any one of claims 2 to 11, wherein the determination unit (15), in a case where the representative moving direction is plural, determines the parameter of which the second motion feature amount in a direction in which the value of the temporal change amount of the motion data is the largest among the representative directions is equal to or more than the second threshold.

13. The information processing system according to any one of claims 2 to 12, wherein the determination unit (15), in a case where the type of the motion is a direction change, determines the parameter of which a value of motion data in a direction in which a motion amount is the smallest among the specified directions is positive.

14. The information processing system according to any one of claims 2 to 13, wherein the determination unit (15), in a case where the type of the motion is straight walking, determines the parameter in which a direction in which the motion amount is the largest among directions other than the specified directions is a rotation direction around a vertical axis orthogonal to a horizontal plane.

15. An information processing method for causing a computer to execute processing for:

receiving original motion data regarding a motion from a sensor for measuring the motion;
extracting (S102), from the original motion data, motion data of one or more first motion sections in which a motion to be detected is indicated, calculating a value of a temporal change amount of the motion data as a first motion feature amount and extracting one or more second motion sections in which the first motion feature amount is equal to or more than a first extraction threshold from the one or more first motion sections;
determining (S104), for each of the one or more second motion sections, a mixing degree indicating a number of representative moving directions of the motion to be detected;
determining (S105), for each of the one or more second motion sections, a representative moving direction based on the mixing degree; and
determining (S106) a parameter used for conversion of the motion data, of which a second motion feature amount, indicating a value of a temporal change amount of the motion data in the representative moving direction after conversion is equal to or more than a second threshold.

## Patentansprüche

1. **1.** Informationsverarbeitungseinrichtung (1), umfassend:

   eine Kommunikationseinheit (11), die zum Empfangen ursprünglicher Bewegungsdaten in Bezug auf eine Bewegung von einem Sensor (4) zum Messen der Bewegung ausgelegt ist;
   eine Extraktionseinheit (12), die zum Extrahieren, aus den ursprünglichen Bewegungsdaten, von Bewegungsdaten von einem oder mehreren ersten Bewegungsabschnitten, in denen eine zu detektierende Bewegung angegeben ist, Berechnen eines Werts einer Zeitänderungsgröße der Bewegungsdaten als eine erste Bewegungsmerkmalgröße und Extrahieren von einem oder mehreren zweiten Bewegungsabschnitten, in denen die erste Bewegungsmerkmalgröße gleich oder größer als eine erste Extraktionsschwelle ist, aus dem einen oder den mehreren ersten Bewegungsabschnitten ausgelegt ist;
   eine Mischgradbestimmungseinheit (13), die zum Bestimmen,
   für jeden von dem einen oder den mehreren zweiten Bewegungsabschnitten, eines Mischgrads, der eine Anzahl von repräsentativen Bewegungsrichtungen der zu detektierenden Bewegung angibt, ausgelegt ist;
   eine Spezifikationseinheit (14), die zum Bestimmen, für jeden von dem einen oder den mehreren zweiten Bewegungsabschnitten, einer repräsentativen Bewegungsrichtung basierend auf dem Mischgrad ausgelegt ist; und
   eine Bestimmungseinheit (15), die zum Bestimmen eines Parameters ausgelegt ist, der zur Umwandlung der Bewegungsdaten verwendet wird, wobei eine zweite Bewegungsmerkmalgröße, die einen Wert einer Zeitänderungsgröße der Bewegungsdaten in der repräsentativen Bewegungsrichtung nach der Umwandlung angibt, gleich oder größer als eine zweite Schwelle ist.

2. **2.** Informationsverarbeitungssystem, umfassend:

   einen Sensor (4), der zum Messen einer Bewegung ausgelegt ist; und
   eine Informationsverarbeitungseinrichtung nach Anspruch 1.

3. **3.** Informationsverarbeitungssystem nach Anspruch 2, wobei
   die Extraktionseinheit (12) einen Algorithmus zum Extrahieren der Bewegungsdaten von einem oder mehreren ersten Bewegungsabschnitten erhält und, vom Algorithmus, eine Extraktionsschwelle als die erste Schwelle erhält.

4. **4.** Informationsverarbeitungssystem nach Anspruch 2 oder 3, wobei
   die Bestimmungseinheit (15) den Parameter bestimmt, der zur Umwandlung der Bewegungsdaten verwendet wird, wobei die zweite Bewegungsmerkmalgröße in der repräsentativen Bewegungsrichtung maximiert wird.

5. **5.** Informationsverarbeitungssystem nach einem der Ansprüche 2 bis 4, wobei
   die Extraktionseinheit (12) einen Verbundwert der Bewegungsdaten für jede der Bewegungsrichtungen als die erste Bewegungsmerkmalgröße verwendet.

6. **6.** Informationsverarbeitungssystem nach einem der Ansprüche 2 bis 5, wobei
   die Mischgradbestimmungseinheit (13) den Mischgrad durch Verwenden einer Hauptkomponentenanalyse bestimmt.

7. **7.** Informationsverarbeitungssystem nach einem der Ansprüche 2 bis 6, wobei
   die Informationsverarbeitungseinrichtung (1) eine Umwandlungseinheit einschließt, die die Bewegungsdaten durch Verwenden des bestimmten Parameters umwandelt.

8. **8.** Informationsverarbeitungssystem nach einem der Ansprüche 2 bis 7, wobei
   die Bestimmungseinheit (15) den Parameter bestimmt, wobei die zweite Bewegungsmerkmalgröße gleich oder größer als die zweite Schwelle für jeden Zeitraum, basierend auf dem Typ der Bewegung bestimmt, ist.

9. **9.** Informationsverarbeitungssystem nach einem der Ansprüche 2 bis 8, wobei
   die Informationsverarbeitungseinrichtung (1) eine erste Abnormalitätsbestimmungseinheit (18) einschließt, die eine Zustandsabnormalität des Sensors basierend auf einer Abweichung der Bewegungsdaten bestimmt.

10. **10.** Informationsverarbeitungssystem nach einem der Ansprüche 2 bis 9, wobei
   die Bestimmungseinheit (15) den Parameter bestimmt, wobei die zweite Bewegungsmerkmalgröße einen positiven Wert gleich oder größer als die zweite Schwelle in einem Fall aufweist, in dem der Typ der Bewegung Gehen ist.

**11.** Informationsverarbeitungssystem nach einem der Ansprüche 2 bis 10, wobei
die Bestimmungseinheit (15) den Parameter in einem ersten Bewegungsabschnitt aus Parameterkandidaten basierend auf einem Unterschied zwischen dem Parameterkandidaten im ersten Bewegungsabschnitt der Bewegungsdaten und einem Parameter, der für einen einzelnen oder eine Vielzahl von zweiten Bewegungsabschnitten bestimmt wird, die eine vorbestimmte Zeit vor oder nach dem ersten Bewegungsabschnitt sind, bestimmt.

**12.** Informationsverarbeitungssystem nach einem der Ansprüche 2 bis 11, wobei
die Bestimmungseinheit (15) in einem Fall, in dem die repräsentative Bewegungsrichtung mehrfach ist, den Parameter bestimmt, wobei die zweite Bewegungsmerkmalgröße in einer Richtung, in der der Wert der Zeitänderungsgröße der Bewegungsdaten der größte unter den repräsentativen Richtungen ist, gleich oder größer als die zweite Schwelle ist.

**13.** Informationsverarbeitungssystem nach einem der Ansprüche 2 bis 12, wobei
die Bestimmungseinheit (15) in einem Fall, in dem der Typ der Bewegung eine Richtungsänderung ist, den Parameter bestimmt, wobei ein Wert von Bewegungsdaten in einer Richtung, in der eine Bewegungsgröße die kleinste unter den vorgegebenen Richtungen ist, positiv ist.

**14.** Informationsverarbeitungssystem nach einem der Ansprüche 2 bis 13, wobei
die Bestimmungseinheit (15) in einem Fall, in dem der Typ der Bewegung Geradeausgehen ist, den Parameter bestimmt, in dem eine Richtung, in der die Bewegungsgröße die größte unter anderen Richtungen als den vorgegebenen Richtungen ist, eine Drehrichtung um eine vertikale Achse orthogonal zu einer horizontalen Ebene ist.

**15.** Informationsverarbeitungsverfahren zum Veranlassen eines Computers zum Ausführen einer Verarbeitung zum:

Empfangen ursprünglicher Bewegungsdaten in Bezug auf eine Bewegung von einem Sensor zum Messen der Bewegung;
Extrahieren (S102), aus den ursprünglichen Bewegungsdaten, von Bewegungsdaten von einem oder mehreren ersten Bewegungsabschnitten, in denen eine zu detektierende Bewegung angegeben ist, Berechnen eines Werts einer Zeitänderungsgröße der Bewegungsdaten als eine erste Bewegungsmerkmalgröße und Extrahieren von einem oder mehreren zweiten Bewegungsabschnitten, in denen die erste Bewegungsmerkmalgröße gleich oder größer als eine erste Extraktionsschwelle ist, aus dem einen oder den mehreren ersten Bewegungsabschnitten;
Bestimmen (S104), für jeden von dem einen oder den mehreren zweiten Bewegungsabschnitten, eines Mischgrads, der eine Anzahl von repräsentativen Bewegungsrichtungen der zu detektierenden Bewegung angibt;
Bestimmen (S105), für jeden von dem einen oder den mehreren zweiten Bewegungsabschnitten, einer repräsentativen Bewegungsrichtung basierend auf dem Mischgrad und
Bestimmen (S106) eines Parameters, der zur Umwandlung der Bewegungsdaten verwendet wird, wobei eine zweite Bewegungsmerkmalgröße, die einen Wert einer Zeitänderungsgröße der Bewegungsdaten in der repräsentativen Bewegungsrichtung nach der Umwandlung angibt, gleich oder größer als eine zweite Schwelle ist.

## Revendications

**1.** Appareil de traitement d’informations (1) comprenant :

une unité de communication (11) configurée pour recevoir des données de mouvement d’origine concernant un mouvement en provenance d’un capteur (4) pour mesurer le mouvement ;
une unité d’extraction (12) configurée pour extraire, à partir des données de mouvement d’origine, des données de mouvement d’une ou plusieurs premières sections de mouvement dans lesquelles un mouvement à détecter est indiqué, calculer une valeur d’une quantité de changement temporel des données de mouvement en tant que première quantité caractéristique de mouvement et extraire une ou plusieurs secondes sections de mouvement dans lesquelles la première quantité caractéristique de mouvement est supérieure ou égale à un premier seuil d’extraction à partir des une ou plusieurs premières sections de mouvement ;
une unité de détermination de degré de mélange (13) configurée pour déterminer,
pour chacune des une ou plusieurs secondes sections de mouvement, un degré de mélange indiquant un nombre de directions de déplacement représentatives du mouvement à détecter ;

une unité de spécification (14) configurée pour déterminer, pour chacune des une ou plusieurs secondes sections de mouvement, une direction de déplacement représentative sur la base du degré de mélange ; et
une unité de détermination (15) configurée pour déterminer un paramètre utilisé pour la conversion des données de mouvement, dont une seconde quantité caractéristique de mouvement, indiquant une valeur d'une quantité de changement temporel des données de mouvement dans la direction de déplacement représentative après conversion, est supérieure ou égale à un second seuil.

**2.** Système de traitement d'informations comprenant :

un capteur (4) configuré pour mesurer un mouvement ; et
un appareil de traitement d'informations selon la revendication 1.

**3.** Système de traitement d'informations selon la revendication 2, dans lequel
l'unité d'extraction (12) obtient un algorithme pour extraire les données de mouvement des une ou plusieurs premières sections de mouvement et obtient, à partir de l'algorithme, un seuil d'extraction en tant que premier seuil.

**4.** Système de traitement d'informations selon la revendication 2 ou 3, dans lequel
l'unité de détermination (15) détermine le paramètre utilisé pour la conversion des données de mouvement dont la seconde quantité caractéristique de mouvement dans la direction de déplacement représentative est rendue maximale.

**5.** Système de traitement d'informations selon l'une quelconque des revendications 2 à 4, dans lequel
l'unité d'extraction (12) utilise une valeur composite des données de mouvement pour chacune des directions de déplacement en tant que première quantité caractéristique de mouvement.

**6.** Système de traitement d'informations selon l'une quelconque des revendications 2 à 5, dans lequel
l'unité de détermination de degré de mélange (13) détermine le degré de mélange en utilisant une analyse de composantes principales.

**7.** Système de traitement d'informations selon l'une quelconque des revendications 2 à 6, dans lequel
l'appareil de traitement d'informations (1) inclut une unité de conversion qui convertit les données de mouvement en utilisant le paramètre déterminé.

**8.** Système de traitement d'informations selon l'une quelconque des revendications 2 à 7, dans lequel
l'unité de détermination (15) détermine le paramètre dont la seconde quantité caractéristique de mouvement est supérieure ou égale au second seuil pour chaque période déterminée sur la base du type du mouvement.

**9.** Système de traitement d'informations selon l'une quelconque des revendications 2 à 8, dans lequel
l'appareil de traitement d'informations (1) inclut une première unité de détermination d'anomalie (18) qui détermine une anomalie d'état du capteur sur la base d'une variation des données de mouvement.

**10.** Système de traitement d'informations selon l'une quelconque des revendications 2 à 9, dans lequel
l'unité de détermination (15) détermine le paramètre dont la seconde quantité caractéristique de mouvement présentant une valeur positive est supérieure ou égale au second seuil dans un cas où le type du mouvement est la marche.

**11.** Système de traitement d'informations selon l'une quelconque des revendications 2 à 10, dans lequel
l'unité de détermination (15) détermine le paramètre dans une première section de mouvement parmi des paramètres candidats sur la base d'une différence entre le paramètre candidat dans la première section de mouvement des données de mouvement et un paramètre déterminé pour une seule ou une pluralité de secondes sections de mouvement qui correspond à un instant prédéterminé avant ou après la première section de mouvement.

**12.** Système de traitement d'informations selon l'une quelconque des revendications 2 à 11, dans lequel
l'unité de détermination (15), dans un cas où la direction de déplacement représentative est plurielle, détermine le paramètre dont la seconde quantité caractéristique de mouvement dans une direction dans laquelle la valeur de la quantité de changement temporel des données de mouvement est la plus importante parmi les directions représentatives est supérieure ou égale au second seuil.

**13.** Système de traitement d'informations selon l'une quelconque des revendications 2 à 12, dans lequel l'unité de détermination (15), dans un cas où le type du mouvement est un changement de direction, détermine le paramètre dont une valeur de données de mouvement dans une direction dans laquelle une quantité de mouvement est la plus petite parmi les directions spécifiées est positive.

**14.** Système de traitement d'informations selon l'une quelconque des revendications 2 à 13, dans lequel l'unité de détermination (15), dans un cas où le type du mouvement est la marche en ligne droite, détermine le paramètre selon lequel une direction dans laquelle la quantité de mouvement est la plus importante parmi des directions autres que les directions spécifiées est une direction de rotation autour d'un axe vertical perpendiculaire à un plan horizontal.

**15.** Procédé de traitement d'informations pour amener un ordinateur à exécuter un traitement pour :

recevoir des données de mouvement d'origine concernant un mouvement en provenance d'un capteur pour mesurer le mouvement ;
extraire (S102), des données de mouvement d'origine, des données de mouvement d'une ou plusieurs premières sections de mouvement dans lesquelles un mouvement à détecter est indiqué, calculer une valeur d'une quantité de changement temporel des données de mouvement en tant que première quantité caractéristique de mouvement et extraire une ou plusieurs secondes sections de mouvement dans lesquelles la première quantité caractéristique de mouvement est supérieure ou égale à un premier seuil d'extraction à partir des une ou plusieurs premières sections de mouvement ;
déterminer (S104), pour chacune des une ou plusieurs secondes sections de mouvement, un degré de mélange indiquant un nombre de directions de déplacement représentatives du mouvement à détecter ;
déterminer (S105), pour chacune des une ou plusieurs secondes sections de mouvement, une direction de déplacement représentative sur la base du degré de mélange ; et
déterminer (S106) un paramètre utilisé pour la conversion des données de mouvement, dont une seconde quantité caractéristique de mouvement, indiquant une valeur d'une quantité de changement temporel des données de mouvement dans la direction de déplacement représentative après conversion est supérieure ou égale à un second seuil.

FIG. 1

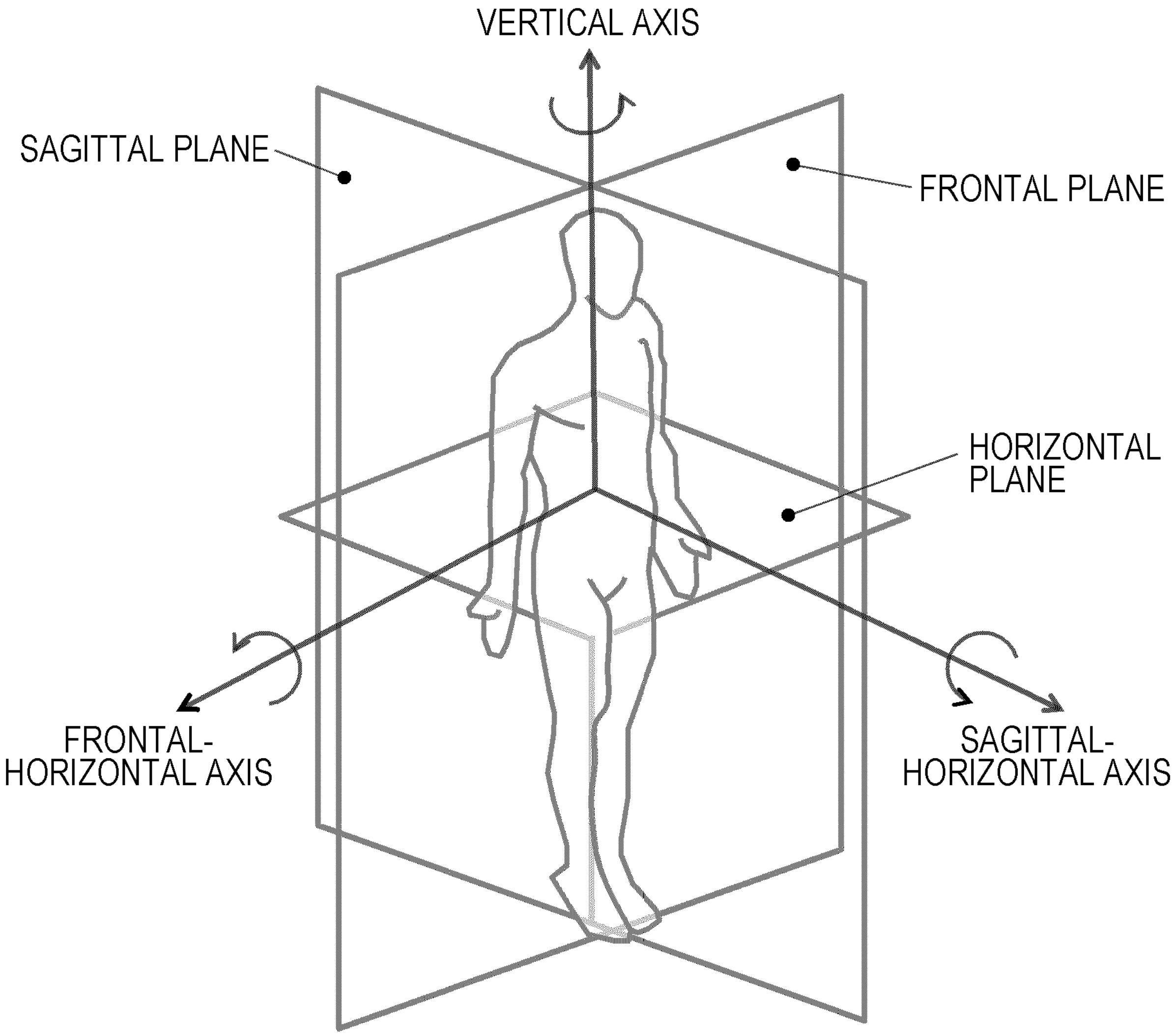
VERTICAL AXIS
SAGITTAL PLANE
FRONTAL PLANE
HORIZONTAL PLANE
FRONTAL-HORIZONTAL AXIS
SAGITTAL-HORIZONTAL AXIS

FIG. 2

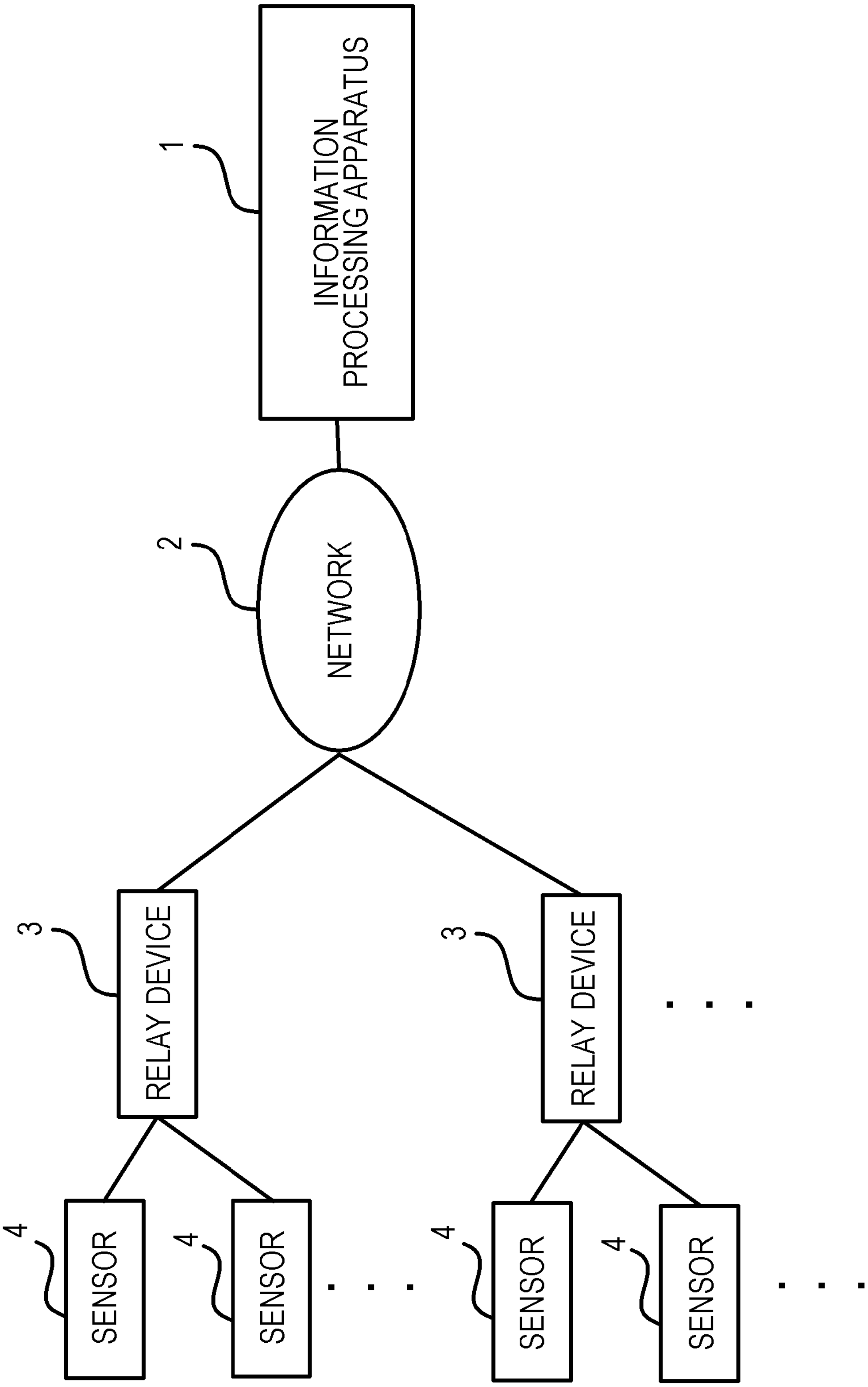

INFORMATION PROCESSING APPARATUS
1
NETWORK
2
RELAY DEVICE
3
RELAY DEVICE
3
SENSOR
4
SENSOR
4
SENSOR
4
SENSOR
4

*FIG. 3*

(a) MOTION DATA AT THE TIME OF WALKING IN A CASE WHERE SENSOR IS NORMALLY ATTACHED

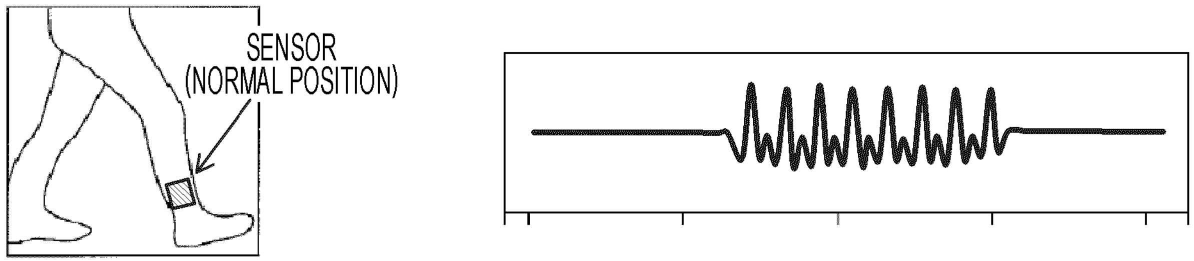


(b) MOTION DATA AT THE TIME OF WALKING IN A CASE WHERE SENSOR IS NOT NORMALLY ATTACHED

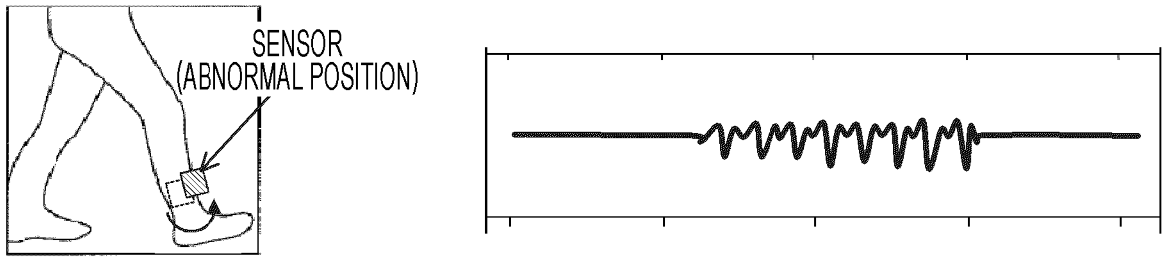

FIG. 4

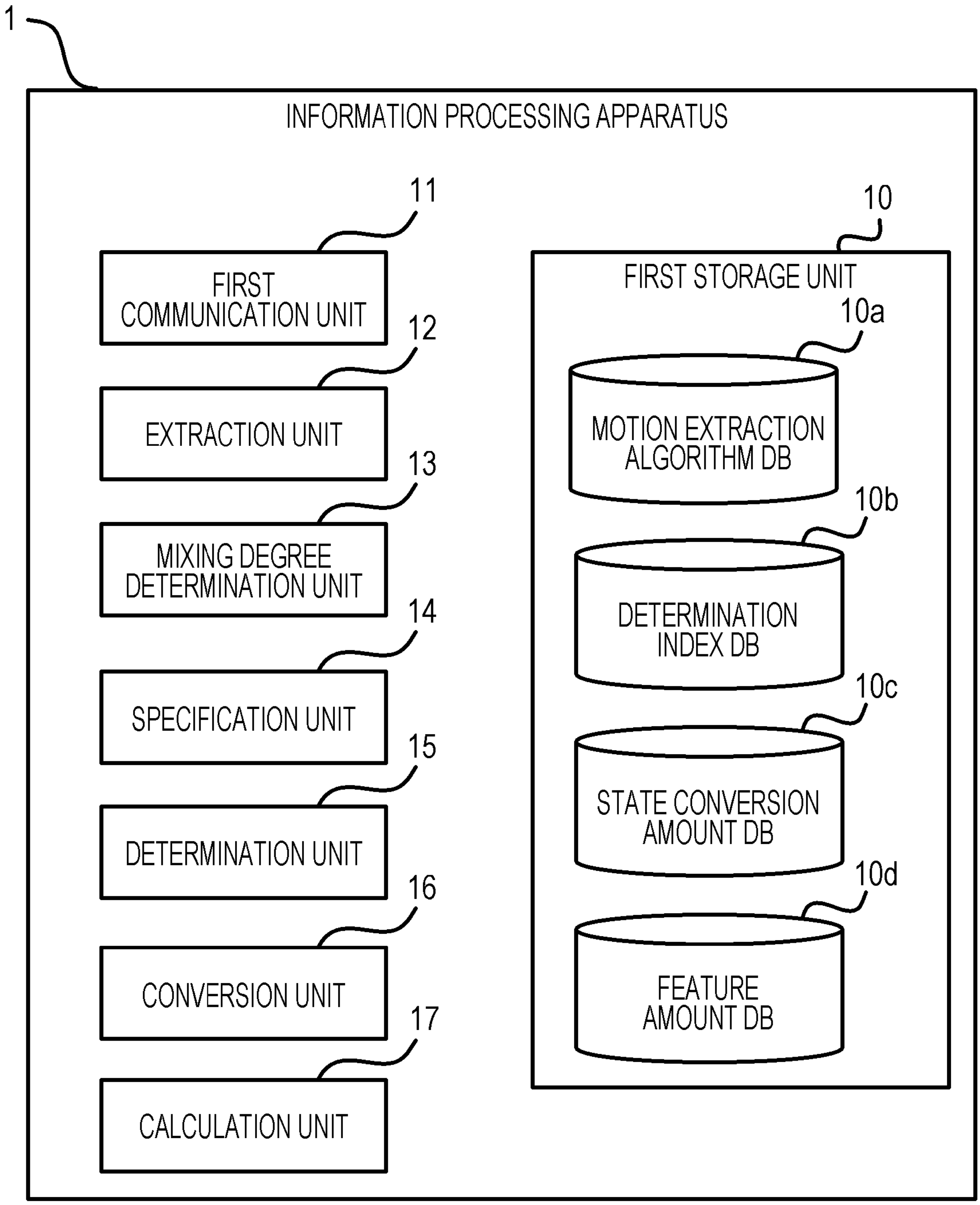
1
INFORMATION PROCESSING APPARATUS
11
FIRST COMMUNICATION UNIT
12
EXTRACTION UNIT
13
MIXING DEGREE DETERMINATION UNIT
14
SPECIFICATION UNIT
15
DETERMINATION UNIT
16
CONVERSION UNIT
17
CALCULATION UNIT
10
FIRST STORAGE UNIT
10a
MOTION EXTRACTION ALGORITHM DB
10b
DETERMINATION INDEX DB
10c
STATE CONVERSION AMOUNT DB
10d
FEATURE AMOUNT DB

FIG. 5

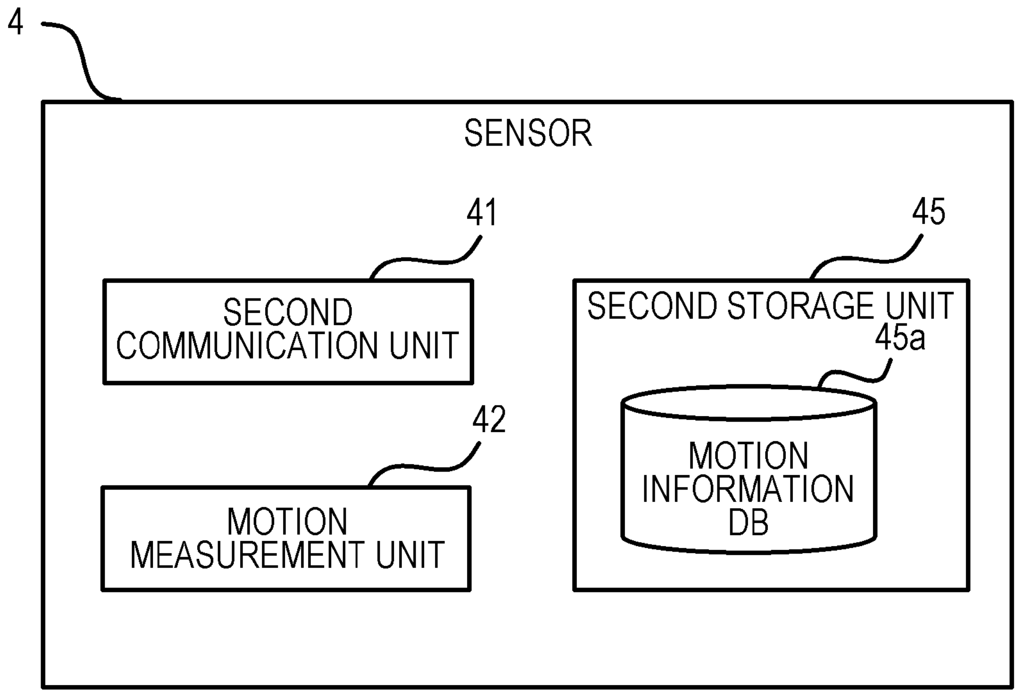
4
SENSOR
41
SECOND COMMUNICATION UNIT
42
MOTION MEASUREMENT UNIT
45
SECOND STORAGE UNIT
45a
MOTION INFORMATION DB

FIG. 6

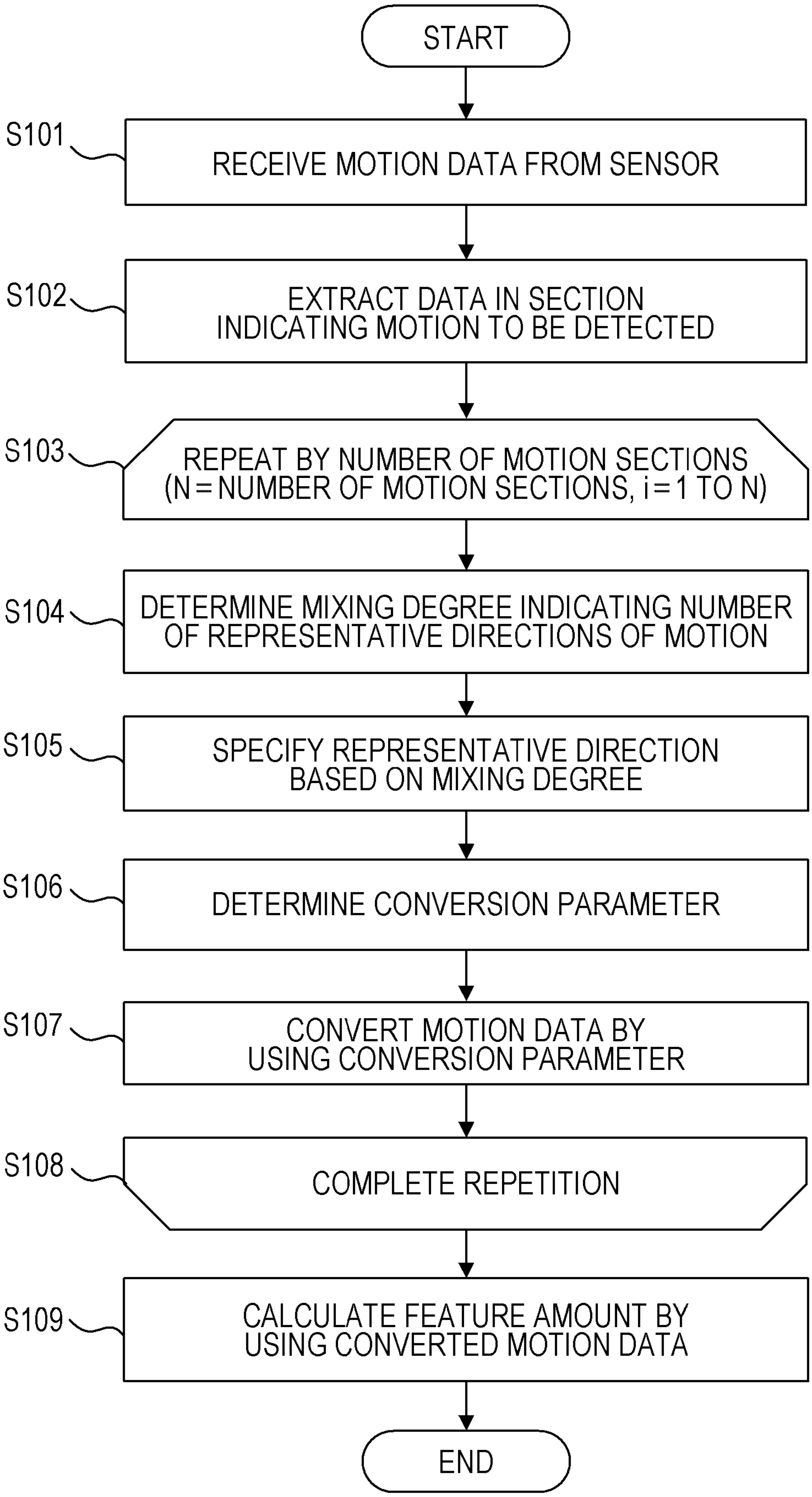
START
S101
RECEIVE MOTION DATA FROM SENSOR
S102
EXTRACT DATA IN SECTION INDICATING MOTION TO BE DETECTED
S103
REPEAT BY NUMBER OF MOTION SECTIONS (N = NUMBER OF MOTION SECTIONS, i = 1 TO N)
S104
DETERMINE MIXING DEGREE INDICATING NUMBER OF REPRESENTATIVE DIRECTIONS OF MOTION
S105
SPECIFY REPRESENTATIVE DIRECTION BASED ON MIXING DEGREE
S106
DETERMINE CONVERSION PARAMETER
S107
CONVERT MOTION DATA BY USING CONVERSION PARAMETER
S108
COMPLETE REPETITION
S109
CALCULATE FEATURE AMOUNT BY USING CONVERTED MOTION DATA
END

FIG. 7

(a) COMPOSITE VALUE OF MOTION DATA OF STRAIGHT WALKING

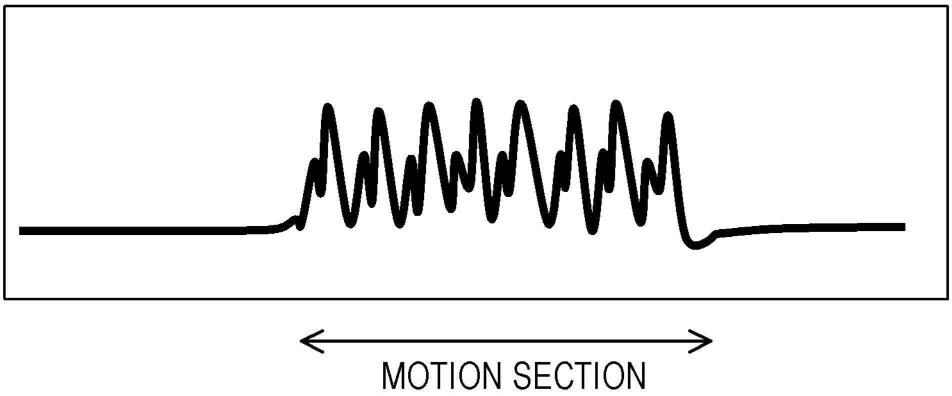


(b) COMPOSITE VALUE OF MOTION DATA OF DIRECTION CHANGE

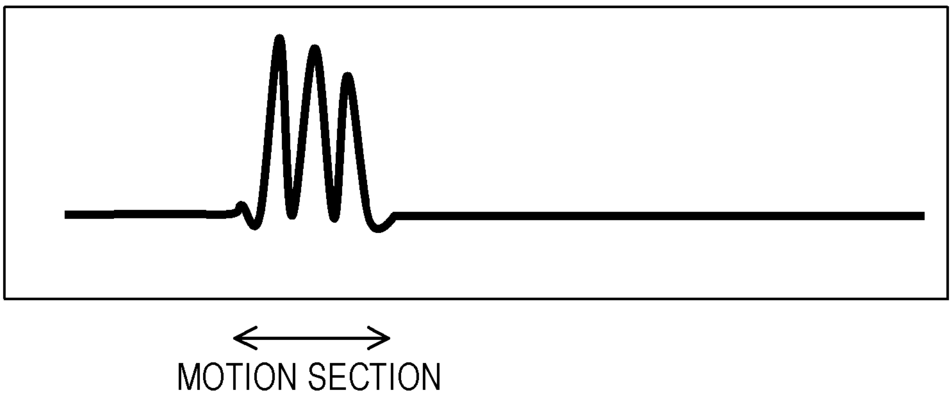

FIG. 8

(a) DETERMINE MIXING DEGREE FOR EACH OF NUMBER OF AXES IN FIRST EXAMPLE

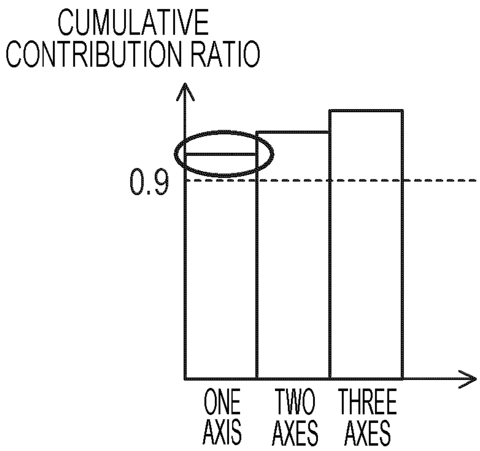


(b) DETERMINE MIXING DEGREE FOR EACH OF NUMBER OF AXES IN SECOND EXAMPLE

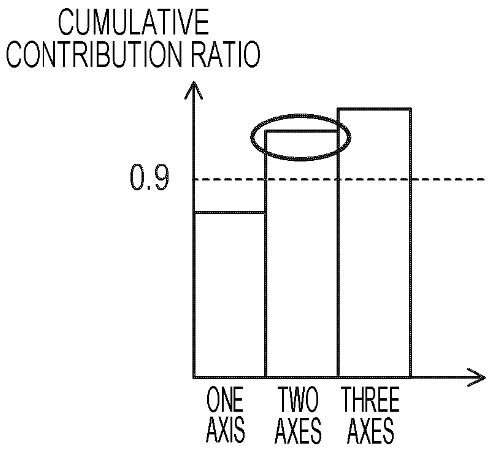


(c) DETERMINE MIXING DEGREE FOR EACH OF NUMBER OF AXES IN THIRD EXAMPLE

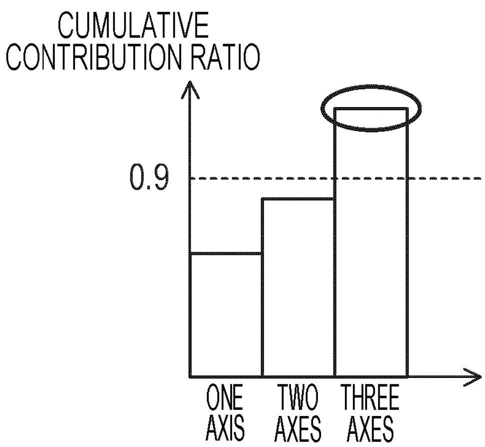

# FIG. 9

(a) MOTION CONVERSION IN A CASE WHERE REPRESENTATIVE DIRECTION IS SINGLE DIRECTION

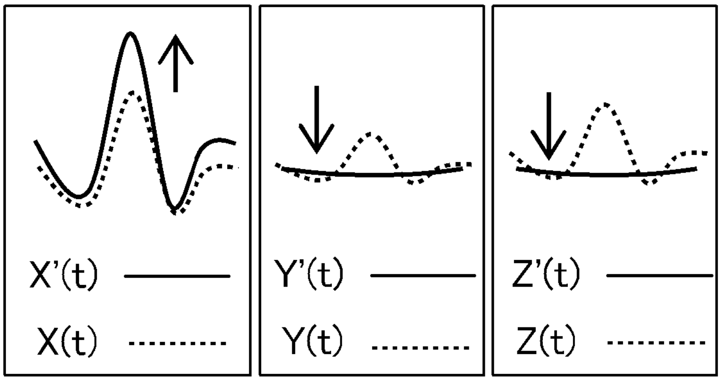


(b) MOTION CONVERSION IN A CASE WHERE REPRESENTATIVE DIRECTION INCLUDES TWO DIRECTIONS

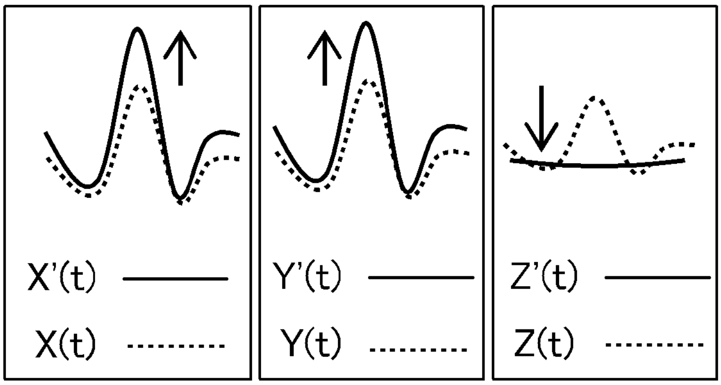


(c) MOTION CONVERSION IN A CASE WHERE REPRESENTATIVE DIRECTION INCLUDES THREE DIRECTIONS

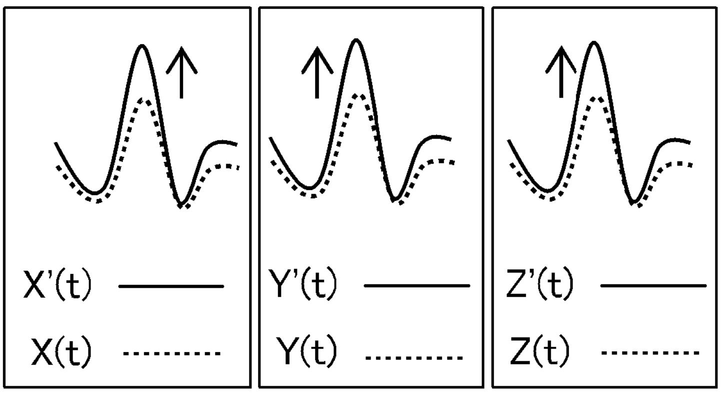

FIG. 10

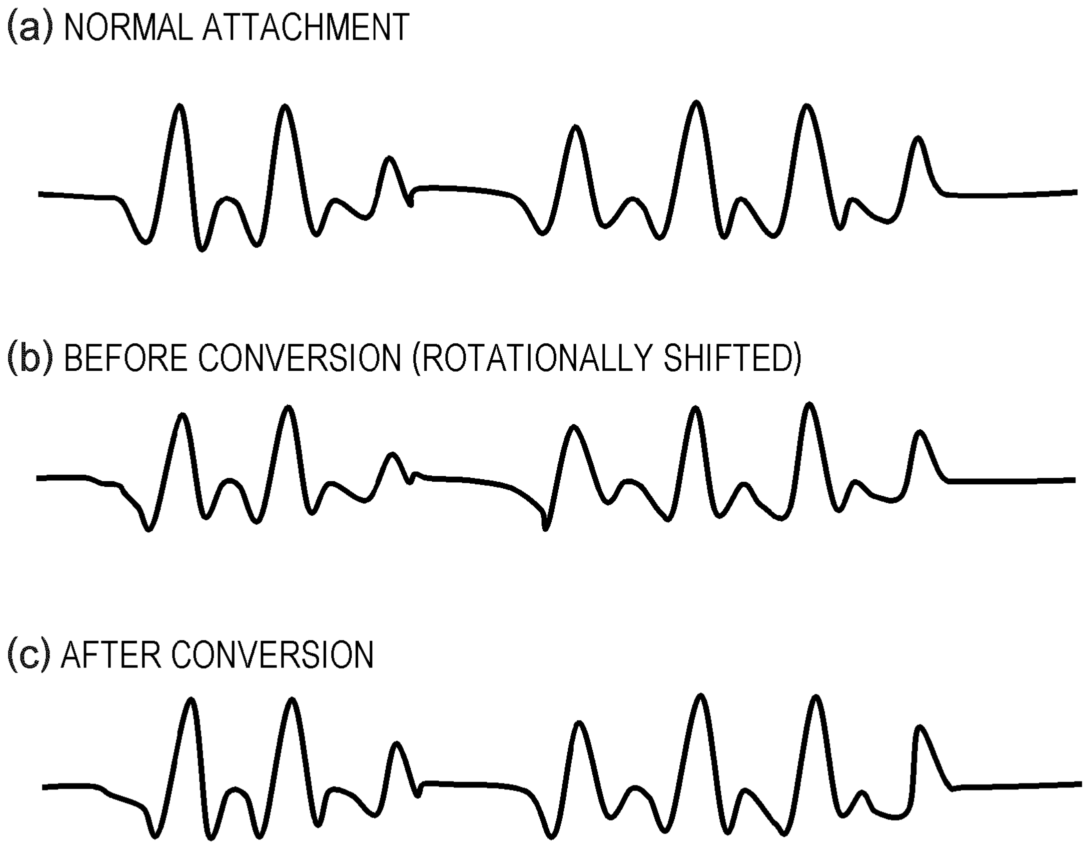
(a) NORMAL ATTACHMENT
(b) BEFORE CONVERSION (ROTATIONALLY SHIFTED)
(c) AFTER CONVERSION

FIG. 11

(a)

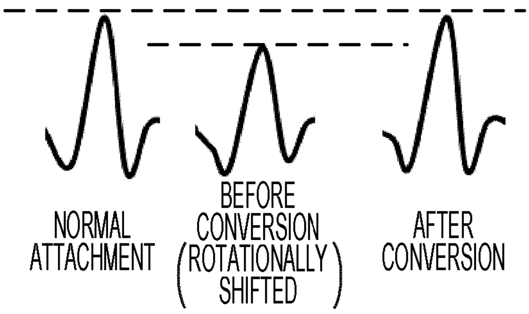
NORMAL ATTACHMENT
BEFORE CONVERSION (ROTATIONALLY SHIFTED)
AFTER CONVERSION

(b)

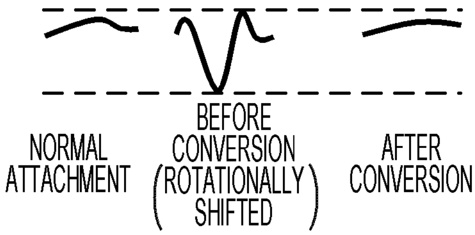
NORMAL ATTACHMENT
BEFORE CONVERSION (ROTATIONALLY SHIFTED)
AFTER CONVERSION

FIG. 12

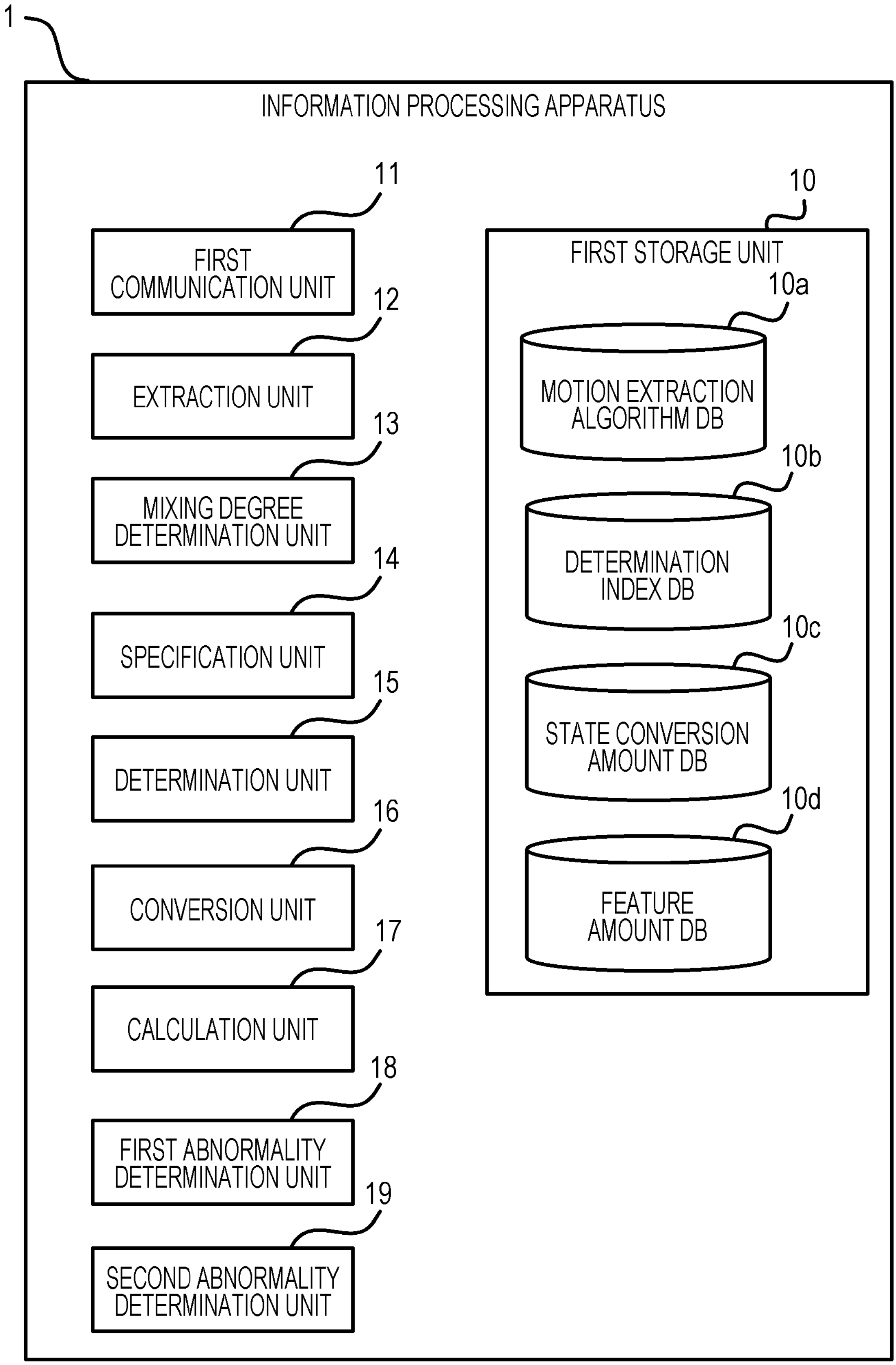
1
INFORMATION PROCESSING APPARATUS
11
FIRST COMMUNICATION UNIT
12
EXTRACTION UNIT
13
MIXING DEGREE DETERMINATION UNIT
14
SPECIFICATION UNIT
15
DETERMINATION UNIT
16
CONVERSION UNIT
17
CALCULATION UNIT
18
FIRST ABNORMALITY DETERMINATION UNIT
19
SECOND ABNORMALITY DETERMINATION UNIT
10
FIRST STORAGE UNIT
10a
MOTION EXTRACTION ALGORITHM DB
10b
DETERMINATION INDEX DB
10c
STATE CONVERSION AMOUNT DB
10d
FEATURE AMOUNT DB

FIG. 13

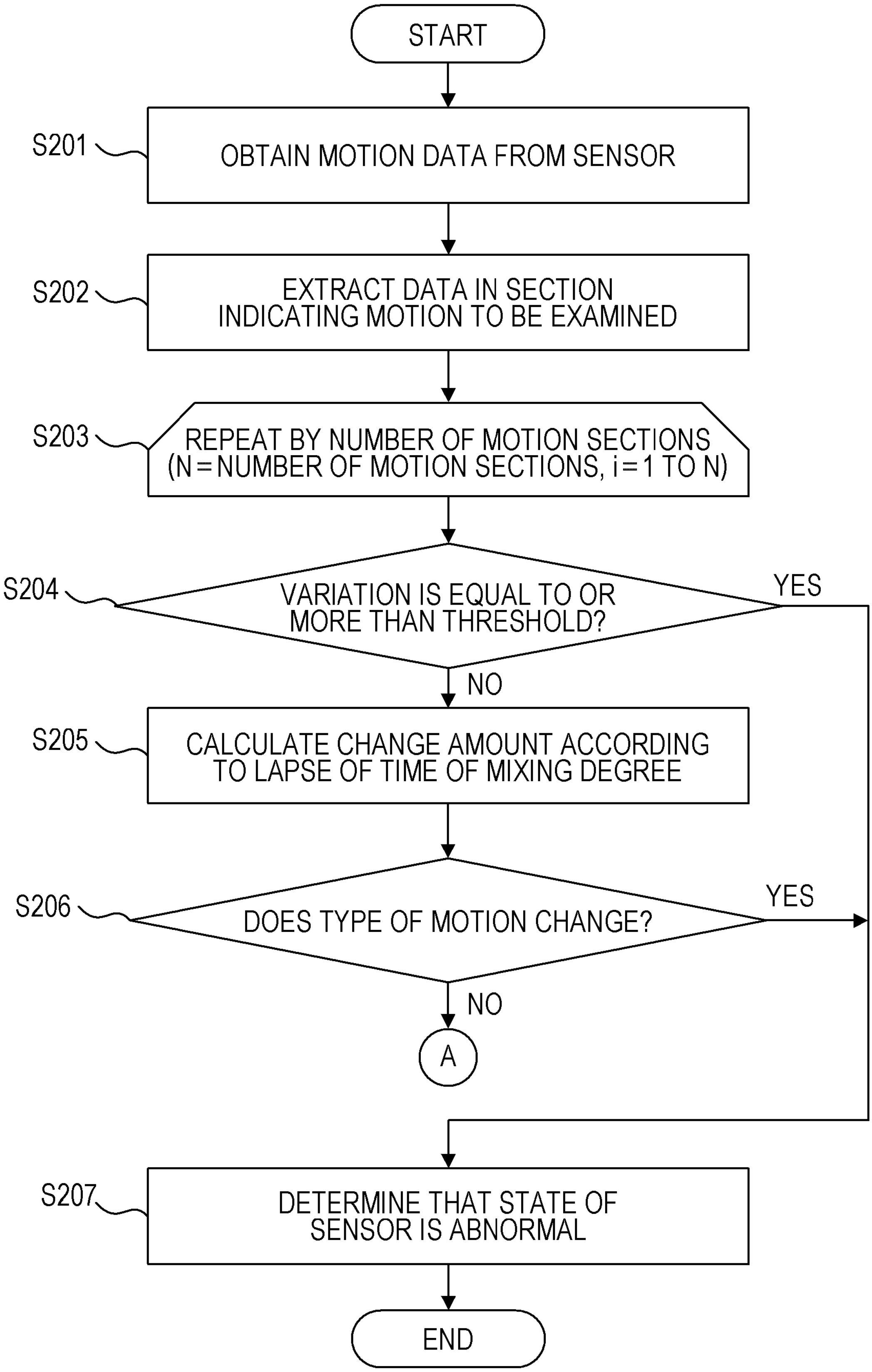
START
S201
OBTAIN MOTION DATA FROM SENSOR
S202
EXTRACT DATA IN SECTION INDICATING MOTION TO BE EXAMINED
S203
REPEAT BY NUMBER OF MOTION SECTIONS (N=NUMBER OF MOTION SECTIONS, i=1 TO N)
S204
VARIATION IS EQUAL TO OR MORE THAN THRESHOLD?
YES
NO
S205
CALCULATE CHANGE AMOUNT ACCORDING TO LAPSE OF TIME OF MIXING DEGREE
S206
DOES TYPE OF MOTION CHANGE?
YES
NO
A
S207
DETERMINE THAT STATE OF SENSOR IS ABNORMAL
END

FIG. 14

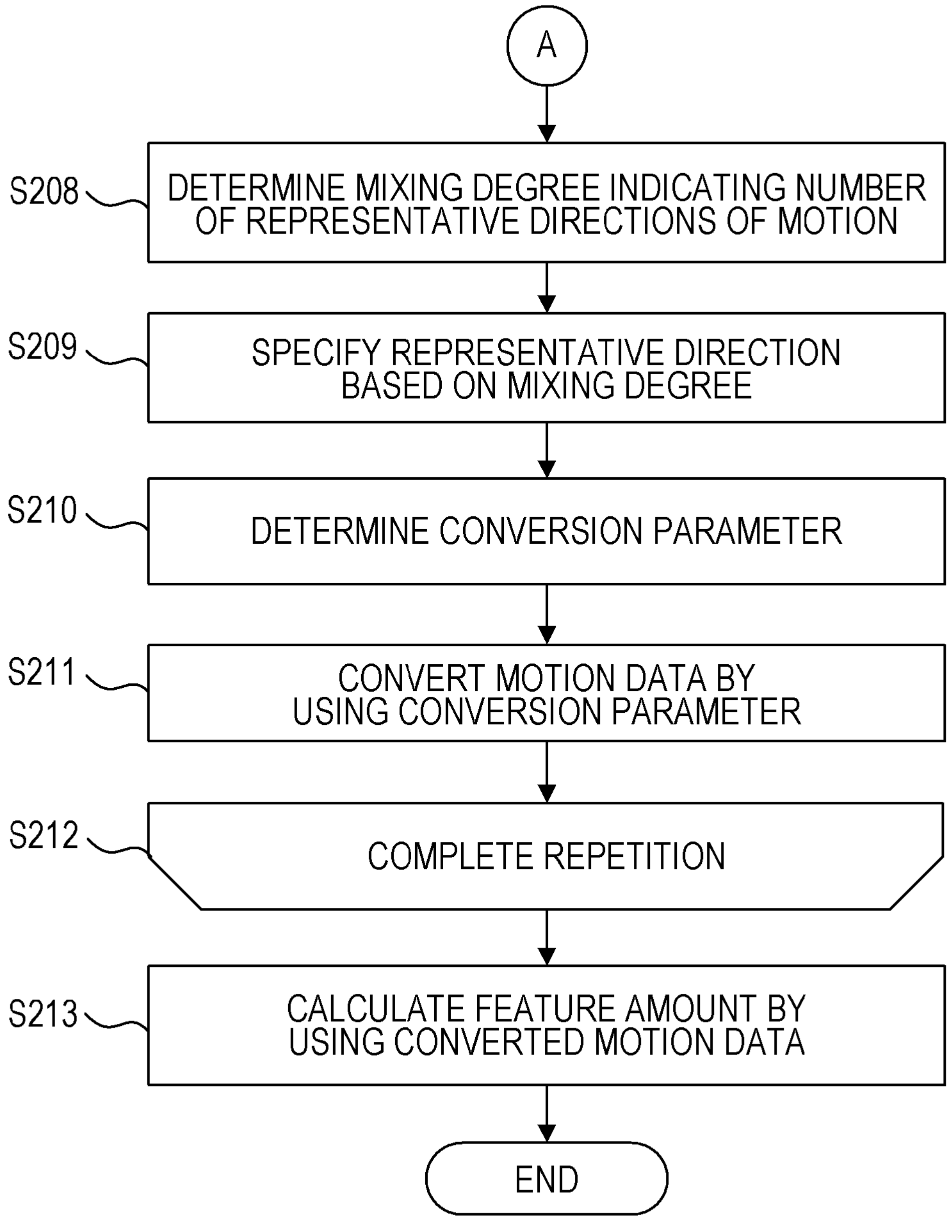
A
S208
DETERMINE MIXING DEGREE INDICATING NUMBER OF REPRESENTATIVE DIRECTIONS OF MOTION
S209
SPECIFY REPRESENTATIVE DIRECTION BASED ON MIXING DEGREE
S210
DETERMINE CONVERSION PARAMETER
S211
CONVERT MOTION DATA BY USING CONVERSION PARAMETER
S212
COMPLETE REPETITION
S213
CALCULATE FEATURE AMOUNT BY USING CONVERTED MOTION DATA
END

FIG. 15

(a) MOTION DATA AT THE TIME OF WALKING IN A CASE WHERE SENSOR IS FIXED

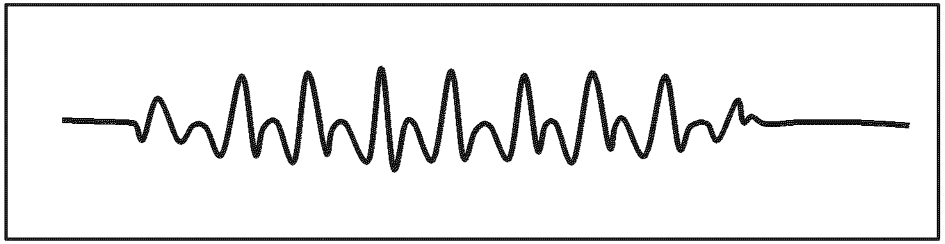

(b) MOTION DATA AT THE TIME OF WALKING IN A CASE WHERE SENSOR IS NOT FIXED

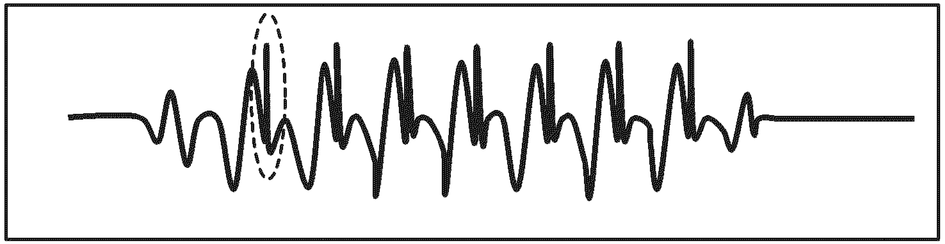

FIG. 16

(a) MOTION DATA OF LEG IN A CASE WHERE OPERATION POSITION IS GRADUALLY CHANGED (FRONTAL-HORIZONTAL AXIS ROTATION DIRECTION)

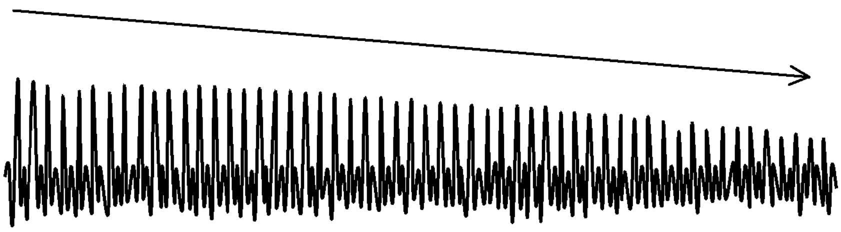

(b) MOTION DATA OF LEG IN A CASE WHERE OPERATION POSITION IS GRADUALLY CHANGED (SAGITTAL-HORIZONTAL AXIS ROTATION DIRECTION)

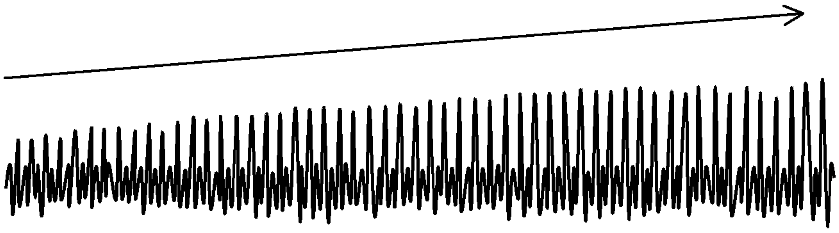

FIG. 17

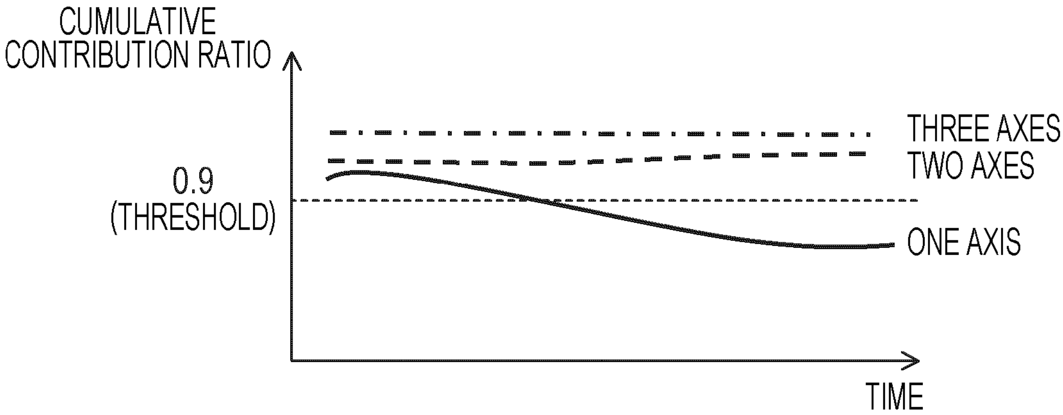
CUMULATIVE
CONTRIBUTION RATIO
0.9
(THRESHOLD)
THREE AXES
TWO AXES
ONE AXIS
TIME

FIG. 18

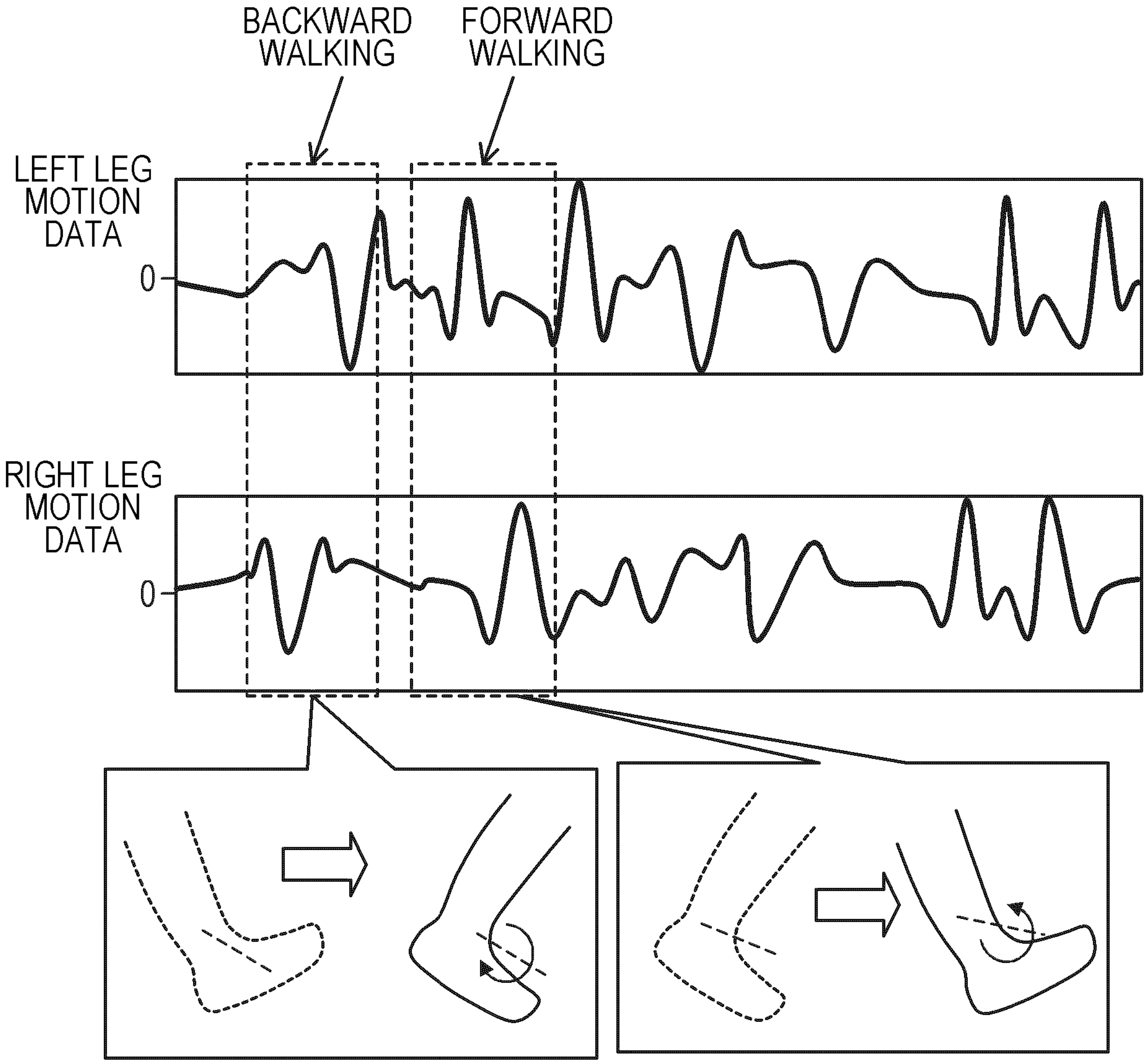
BACKWARD WALKING
FORWARD WALKING
LEFT LEG MOTION DATA
0
RIGHT LEG MOTION DATA
0

FIG. 19

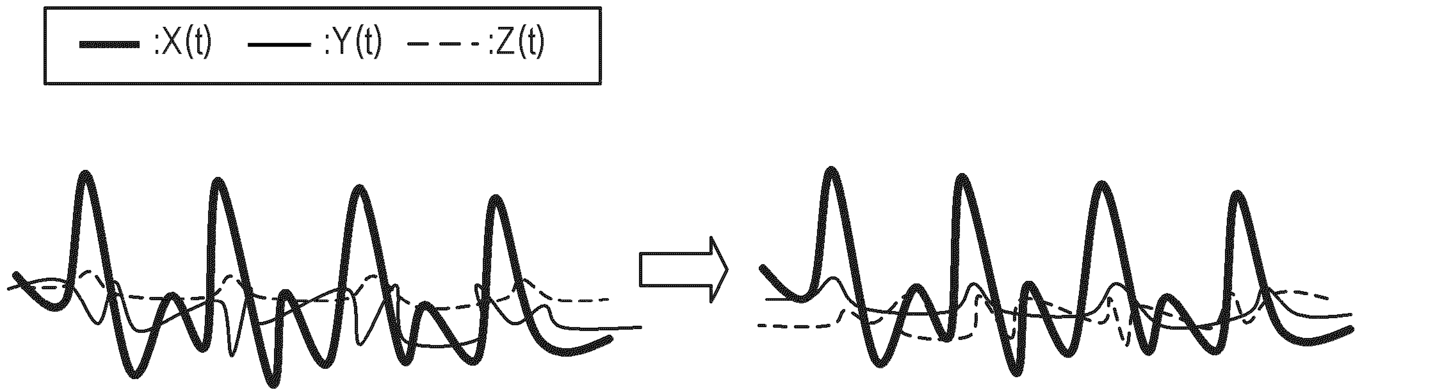


CONVERSION PARAMETER IN SECTION $\tau$:
$\theta = (150, 60, 240)$

CONVERSION PARAMETER IN SECTION $\tau+1$:
$\theta = (150, 240, 60)$

FIG. 20

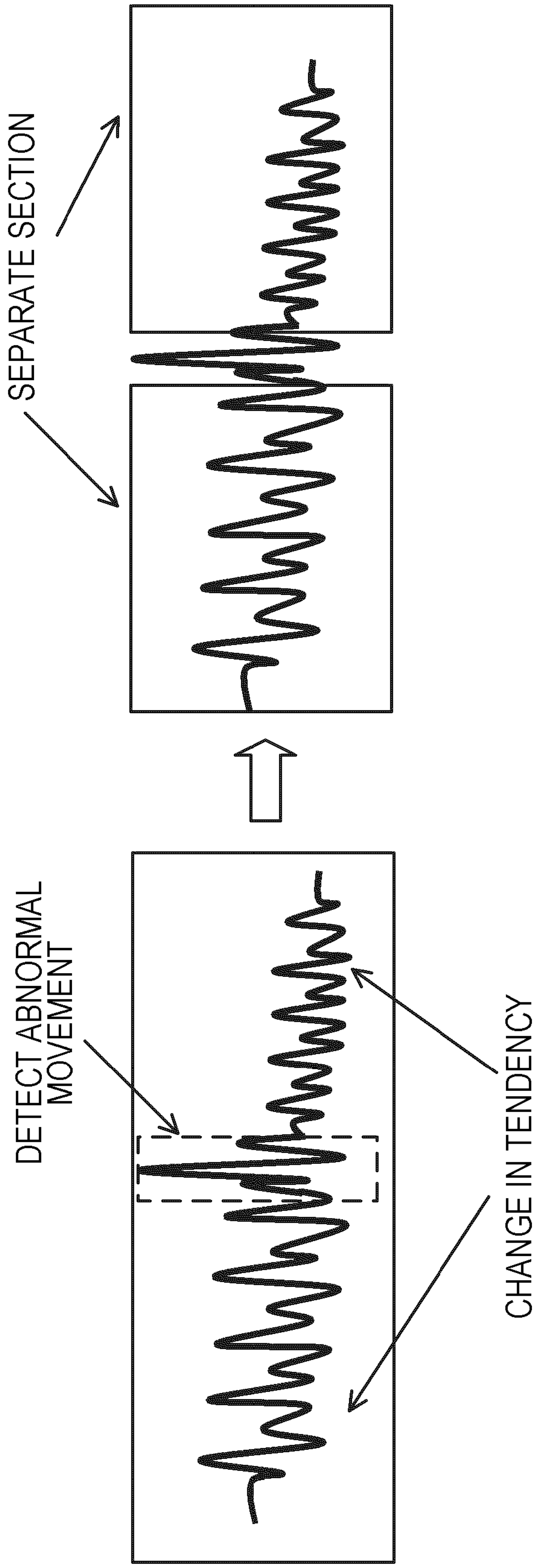
SEPARATE SECTION
DETECT ABNORMAL MOVEMENT
CHANGE IN TENDENCY

FIG. 21

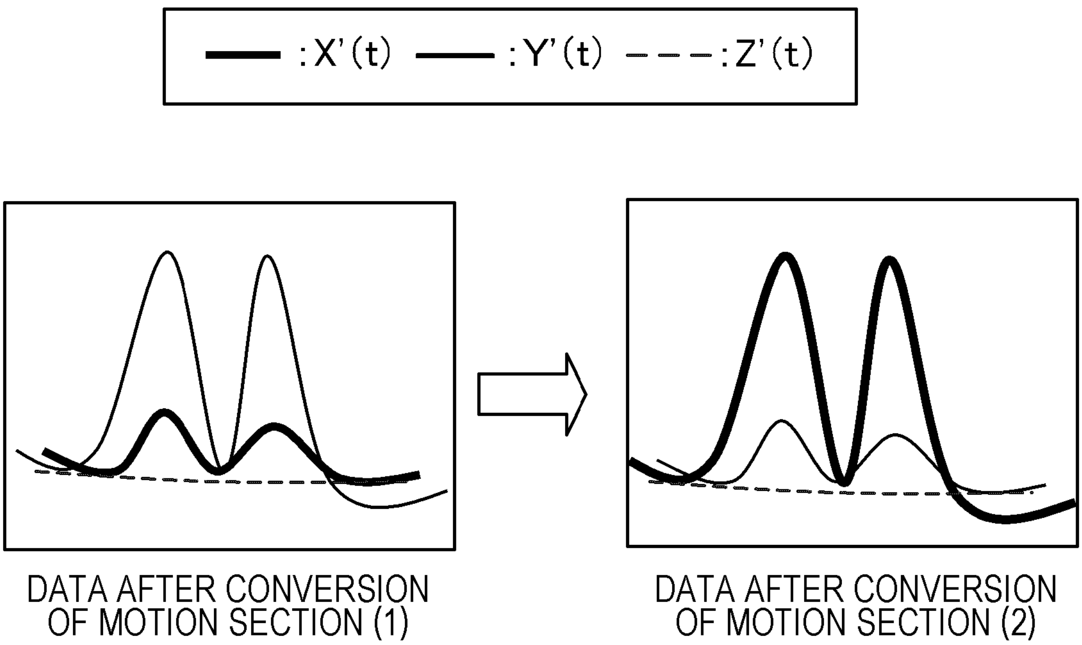
: X'(t)
: Y'(t)
: Z'(t)
DATA AFTER CONVERSION OF MOTION SECTION (1)
DATA AFTER CONVERSION OF MOTION SECTION (2)

# FIG. 22

(a) MOTION DATA IN A CASE WHERE RIGHT TURN IS NORMALLY CONVERTED

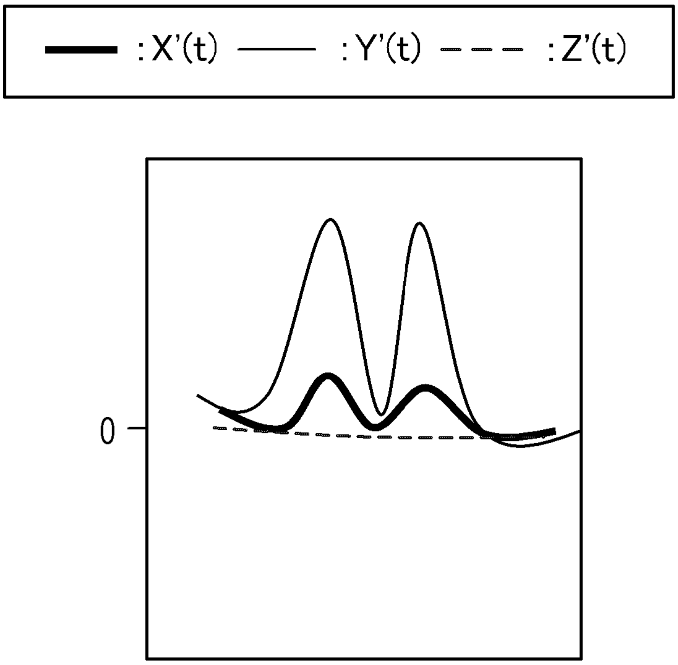


(b) MOTION DATA IN A CASE WHERE RIGHT TURN IS NOT NORMALLY CONVERTED

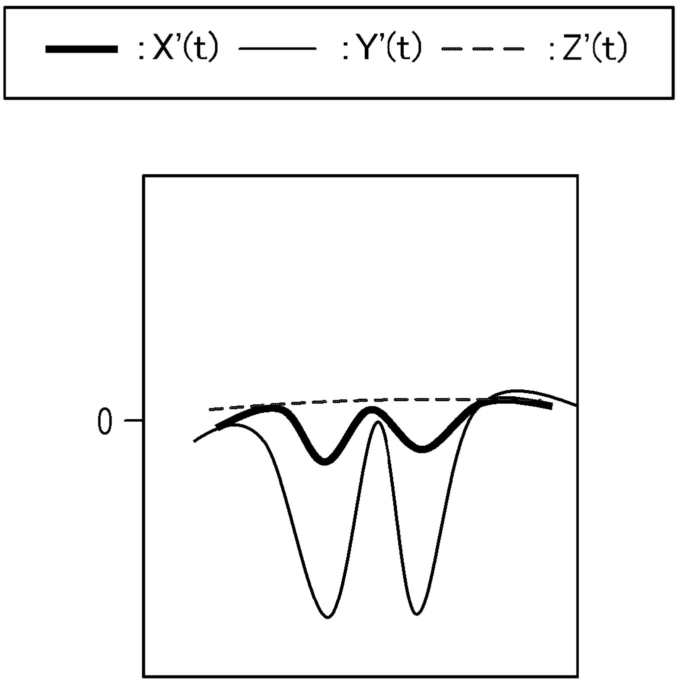

FIG. 23

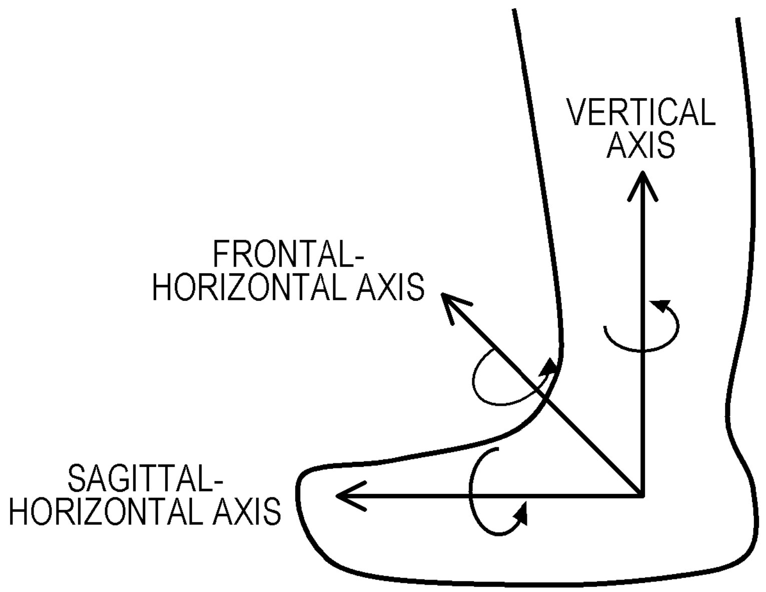
VERTICAL
AXIS
FRONTAL-
HORIZONTAL AXIS
SAGITTAL-
HORIZONTAL AXIS

FIG. 24

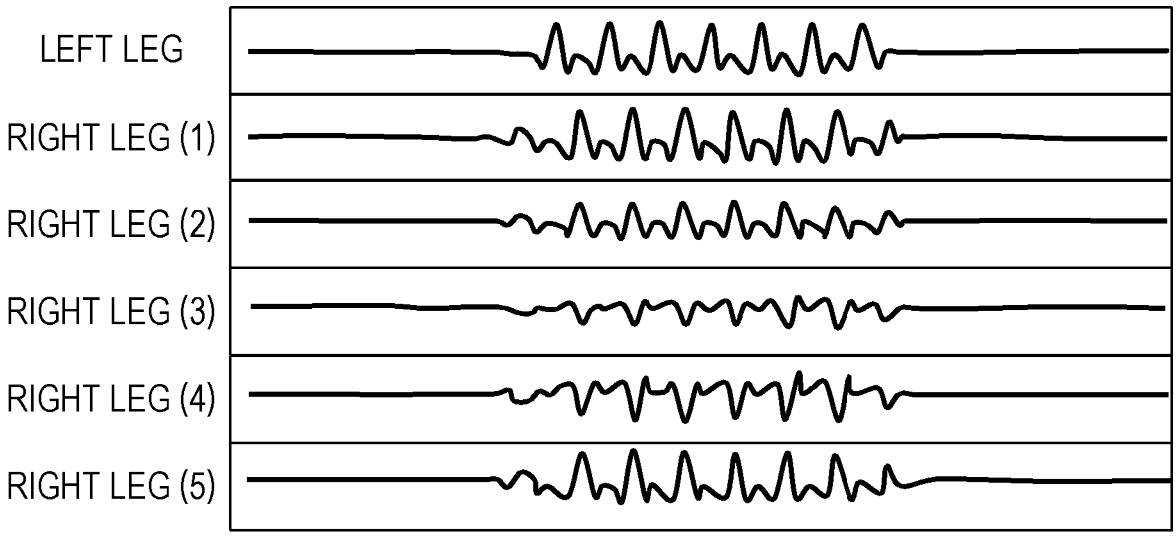
LEFT LEG
RIGHT LEG (1)
RIGHT LEG (2)
RIGHT LEG (3)
RIGHT LEG (4)
RIGHT LEG (5)

FIG. 25

| ALGORITHM No. | PARAMETER 1: PEAK VALUE OF COMPOSITE VALUE [m/s] (THRESHOLD) | PARAMETER 2: PEAK INTERVAL OF COMPOSITE VALUE [ms] (THRESHOLD) | PARAMETER 3: PEAK WIDTH OF COMPOSITE VALUE [ms] (THRESHOLD) | PARAMETER 4: PEAK GRADIENT OF COMPOSITE VALUE (THRESHOLD) | ··· |
|---|---|---|---|---|---|
| 1 | ○ (300) | ○ (50) | × | × | ··· |
| 2 | × | ○ (55) | × | × | ··· |
| ··· | ··· | ··· | ··· | ··· | ··· |

FIG. 26

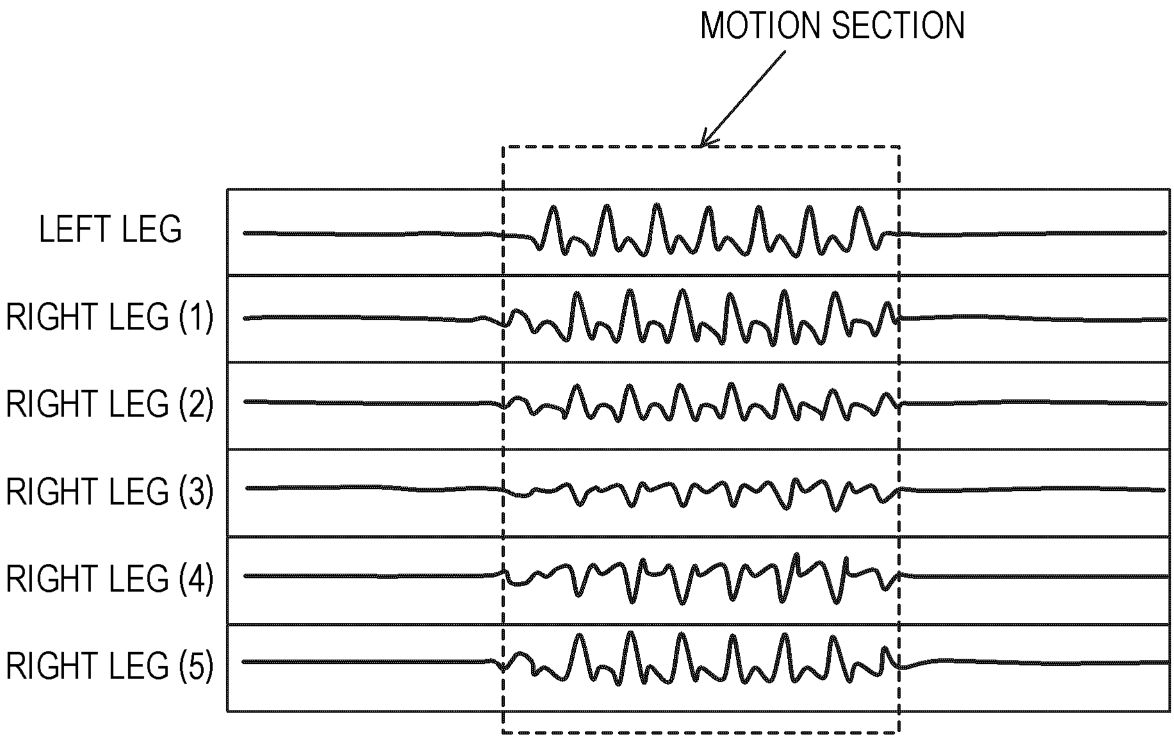
MOTION SECTION
LEFT LEG
RIGHT LEG (1)
RIGHT LEG (2)
RIGHT LEG (3)
RIGHT LEG (4)
RIGHT LEG (5)

FIG. 27

| DEFINITION No | TYPE OF TARGET MOTION | MIXING DEGREE DETERMINATION INDEX | | | |
|---|---|---|---|---|---|
| | | CUMULATIVE CONTRIBUTION RATIO OF MAIN COMPONENT ANALYSIS (THRESHOLD) | RATIO OF MIXING COEFFICIENTS IN INDEPENDENT COMPONENT ANALYSIS (THRESHOLD) | VARIATION IN SENSOR VALUE (THRESHOLD) | ... |
| 1 | STRAIGHT WALKING | 0.9 | - | - | ... |
| 2 | DIRECTION CHANGE | 0.95 | - | - | ... |
| 3 | NONE SPECIFIED | - | 0.7 | - | ... |
| ... | ... | ... | ... | - | ... |

FIG. 28

| DATE AND TIME | MOTION START TIME | MOTION END TIME | STATE CONVERSION AMOUNT | | |
|---|---|---|---|---|---|
| | | | X-AXIS ROTATION AMOUNT | Y-AXIS ROTATION AMOUNT | Z-AXIS ROTATION AMOUNT |
| 2016/03/31 | 20:10:05 | 20:10:15 | 150° | 60° | 240° |
| ... | ... | ... | ... | ... | ... |

FIG. 29

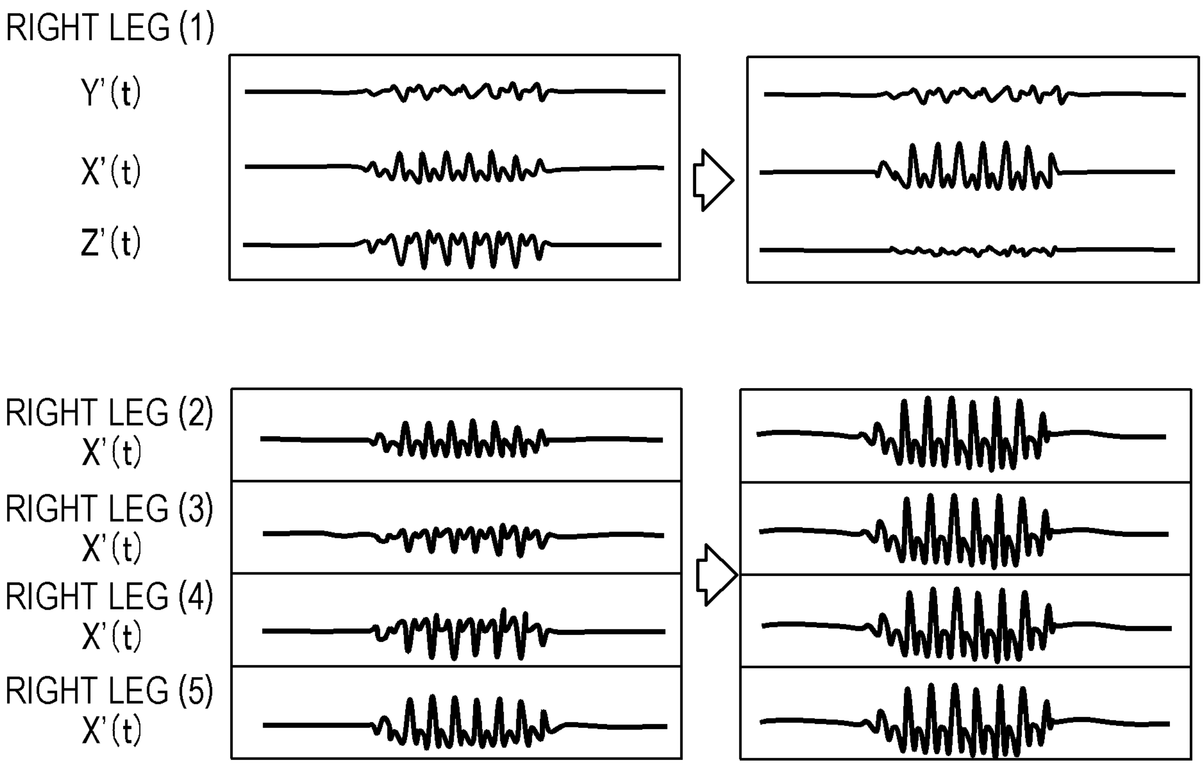
RIGHT LEG (1)
Y'(t)
X'(t)
Z'(t)
RIGHT LEG (2)
X'(t)
RIGHT LEG (3)
X'(t)
RIGHT LEG (4)
X'(t)
RIGHT LEG (5)
X'(t)

*FIG. 30*

| DATE AND TIME | LEFT LEG WALKING SPEED [m/s] | RIGHT LEG WALKING SPEED [m/s] | LEFT LEG STRIDE LENGTH [m] | RIGHT LEG STRIDE LENGTH [m] | ... |
|---|---|---|---|---|---|
| 2016/03/31 | 0.72 | 0.71 | 0.66 | 0.67 | ... |
| ... | ... | | | ... | ... |

FIG. 31

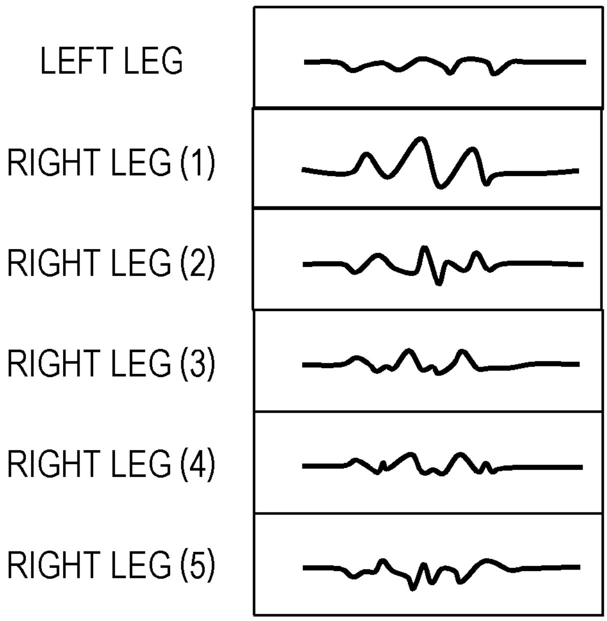
LEFT LEG
RIGHT LEG (1)
RIGHT LEG (2)
RIGHT LEG (3)
RIGHT LEG (4)
RIGHT LEG (5)

FIG. 32

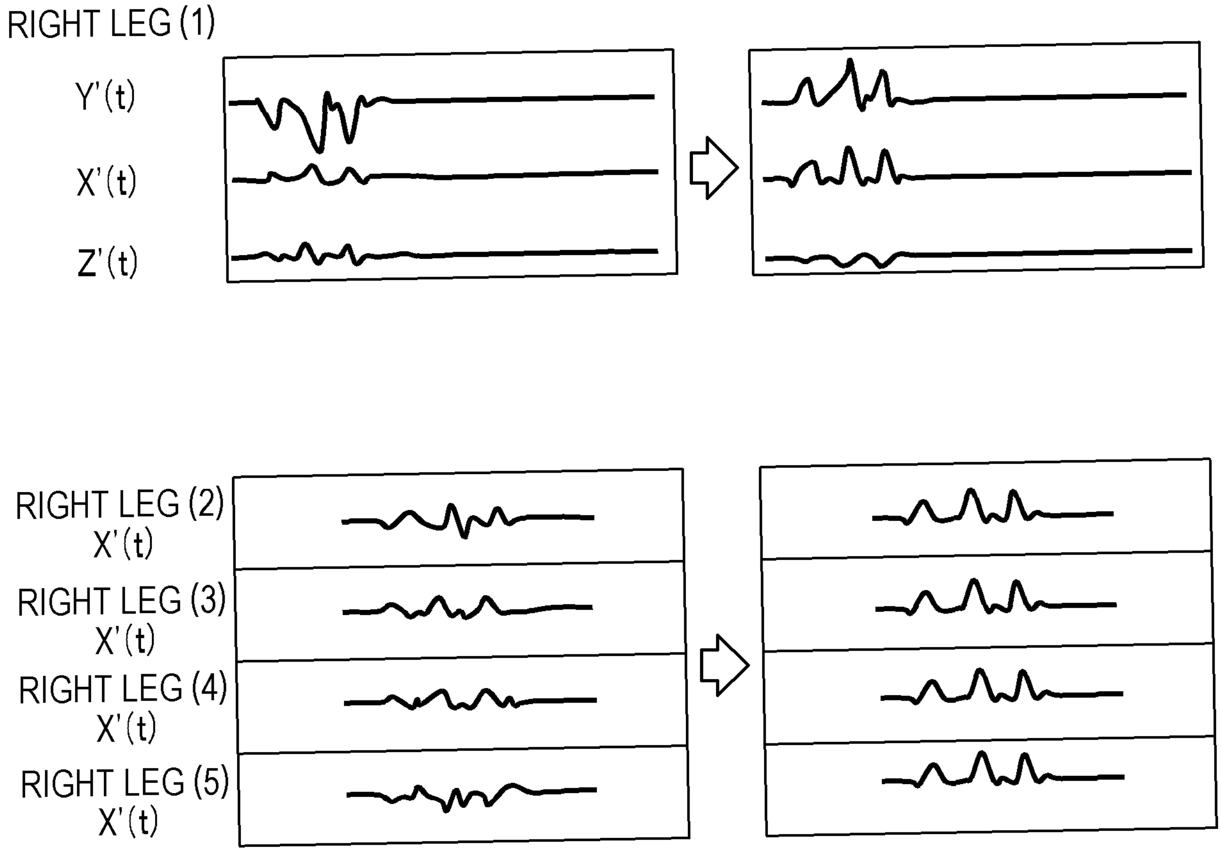
RIGHT LEG (1)
Y'(t)
X'(t)
Z'(t)
RIGHT LEG (2)
X'(t)
RIGHT LEG (3)
X'(t)
RIGHT LEG (4)
X'(t)
RIGHT LEG (5)
X'(t)

FIG. 33

| DATE AND TIME | LEFT LEG ROTATION DIRECTION | RIGHT LEG ROTATION DIRECTION | LEFT LEG ROTATION AMOUNT | RIGHT LEG ROTATION AMOUNT | ... |
|---|---|---|---|---|---|
| 2016/03/31 | RIGHT | RIGHT | 360° | 360° | ... |
| ... | ... | | | ... | ... |

FIG. 34

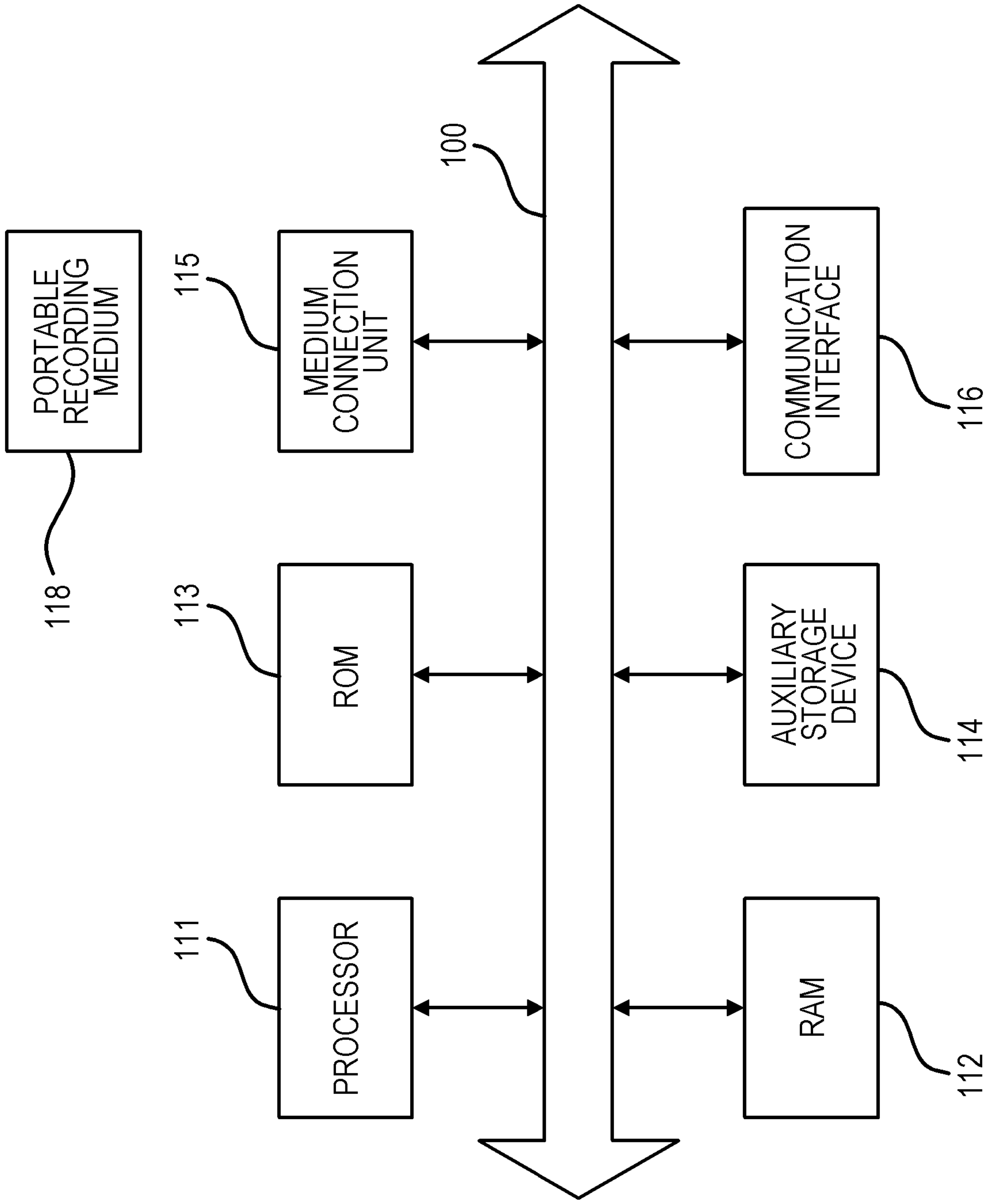
PORTABLE RECORDING MEDIUM
118
MEDIUM CONNECTION UNIT
115
ROM
113
PROCESSOR
111
100
COMMUNICATION INTERFACE
116
AUXILIARY STORAGE DEVICE
114
RAM
112

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013532539 A
- JP 2005172625 A
- WO 2007052631 A
- US 20040064286 A1
- US 20160018225 A1


### Non-patent literature cited in the description


- **FRACCARO et al.** Real-world Gyroscopebased Gait Event Detection and Gait Feature Extraction. *eTELEMED,* 2014
- **VAN LUMMEL et al.** Automated approach for quantifying the repeated sit-to-stand using one body fixed sensor in young and older adults. *Gait & Posture.,* 2013, vol. 38, 153-156